(19) Europäisches Patentamt
European Patent Office
Office européen des brevets

(11) Numéro de publication: **0 547 972 B1**

# (12) FASCICULE DE BREVET EUROPEEN

(45) Date de publication de fascicule du brevet: **22.03.95**

(21) Numéro de dépôt: **92403433.3**

(22) Date de dépôt: **17.12.92**

(51) Int. Cl.6: **C07D 213/75**, A01N 43/00, C07D 277/46, C07D 295/28, C07D 261/14, C07D 285/125, C07D 239/42, C07D 231/40, C07D 307/22

## (54) Dérivés d'amides hétérocicliques et leur utilisation comme pesticides.

(30) Priorité: **19.12.91 GB 9126955**

(43) Date de publication de la demande:
**23.06.93 Bulletin 93/25**

(45) Mention de la délivrance du brevet:
**22.03.95 Bulletin 95/12**

(84) Etats contractants désignés:
**AT BE CH DE DK ES FR GB GR IE IT LI LU NL PT SE**

(56) Documents cités:
**EP-A- 0 269 457**
**EP-A- 0 369 762**
**US-A- 4 950 666**

**CHEMICAL ABSTRACTS. REGISTRY HANDBOOK - NUMBER SECTION. PRINTED ISSUES + MICROFILM 1965, COLUMBUS US**

(73) Titulaire: **ROUSSEL-UCLAF**
**35, Boulevard des Invalides**
**F-75007 Paris (FR)**

(72) Inventeur: **Black, Malcom Henry**
**9 Longfield Gardens**
**Tring,**
**Herts. HP23 4DN (GB)**
Inventeur: **Blade, Robert John**
**23 Fantail Lane**
**Tring,**
**Herts. HP23 4EN (GB)**
Inventeur: **Cockerill, George Stuart**
**33 Hartwort Close,**
**Walnut Tree**
**Milton Keynes, MK7 7LJ (GB)**
Inventeur: **Pulman, David Allen**
**16 Francisco Vista,**
**2041 Francisco Street**
**Berkeley,**
**California CA 94709 (US)**

**EP 0 547 972 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS. REGISTRY HANDBOOK - NUMBER SECTION. PRINTED ISSUES + MICROFILM 1974, COLUMBUS US

CHEMICAL ABSTRACTS. REGISTRY HANDBOOK - NUMBER SECTION. PRINTED ISSUES + MICROFILM 1976, COLUMBUS US

(74) Mandataire: **Vieillefosse, Jean-Claude et al**
**Roussel-Uclaf**
**111, route de Noisy**
**B.P. 9**
**F-93230 Romainville (FR)**

## Description

Cette invention concerne des composés pesticides, des procédés pour les préparer, des compositions les contenant et leur utilisation dans le traitement des nuisibles.

Des amides insaturés possédant une chaîne méthylène de 1 à au moins 10 atomes de carbone, contenant éventuellement au moins un oxygène ou un groupe méthylène supplémentaire, sont connus comme pesticides ou comme insecticides possédant divers groupes terminaux qui englobent dans leur cadre les groupes phényle éventuellement substitués (demande de brevet européen n° 228222, 194764, 225011, demande de brevet japonais n° 57-212150, Meisters et Wailes, Aust. J. Chem. 1966, 19, 1215, Vig et al., J. Ind. Chem. Soc. 1974, 51 (9), 817) ou pyridyle (demande de brevet européen 269457) ou un système bicyclique à noyaux condensés (demandes de brevets européens n° 143593, 228853), un groupe dihalogénovinyle ou un groupe éthynyle éventuellement substitué (demande de brevet européen 228222). EP-A-369762 décrit un groupe interstitiel cycloalkyle reliant le motif diène au groupe terminal.

H.O. Huisman et al, Rev. trav. chim., 77, 97-102 (1958), décrit un groupe de 5-(2,6,6-triméthyl-cyclohexényl)-2,4-pentadiénamides comme insecticides.

US-A-4,950,666 décrit quelques N-(3-pyridyl) amides ayant des propriétés pesticides.

On a maintenant découvert, ce qui est surprenant, que certains nouveaux amides N-hétéro-cycliques dans lesquels l'hétérocycle forme, avec l'azote auquel il se rattache, un groupe

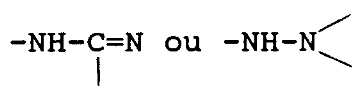

ont une utilité en tant que pesticides ayant des propriétés améliorées par rapport à ceux dans lesquels le groupe amide est un groupe acyclique.

Par conséquent, la présente invention fournit un composé de formule (I):

$Q(CH_2)_a(O)_bQ_1CR_2=CR_3\ CR_4=CR_5\ CX_1NHR_1$

ou un de ses sels, dans lequel Q est un système, éventuellement substitué, monocyclique ou bicyclique à noyaux condensés dans lequel au moins un noyau est aromatique, ou Q est un groupe dihalogénovinyle ou un groupe $R^6C\equiv C$-, dans lequel $R_6$ est un groupe alkyle en $C_1$-$C_4$, tri(alkyl en $C_1$-$C_4$)silyle, halogéno ou un hydrogène;

$Q_1$ est un noyau 1,2-cyclopropyle éventuellement substitué par un ou plusieurs groupes choisis parmi les groupes alkyle en $C_1$-$C_3$, halogéno, halogénoalkyle en $C_1$-$C_3$, alcynyle ou cyano; ou $Q_1$ est $(CH_2)$m, où m = 1 à 7; a = 0 ou 1; b = 0 ou 1;

$R_2$, $R_3$, $R_4$ et $R_5$ sont identiques ou différents, l'un au moins étant un hydrogène et les autres étant indépendamment choisis parmi un hydrogène, un halogène, un groupe alkyle en $C_1$-$C_4$ ou un groupe halogénoalkyle en $C_1$-$C_4$;

$X_1$ est un oxygène ou un soufre;

$R_1$ est un noyau hétérocyclique éventuellement substitué aromatique, partiellement saturé ou saturé, a cinq ou six chainons, contenant au moins un atome d'azote et éventuellement jusqu'à trois autres hétéroatomes choisis parmi, l'azote, l'oxygène ou le soufre, et qui se rattache à l'azote de la fonction amide par un atome d'azote ou par un carbone adjacent à un atome d'azote, ou $R_1$ est le radical 1-azépinyle.

$R_1$ est éventuellement substitué par 1 à 5 substituants choisis parmi un groupe alkyle en $C_1$-$C_4$, alcoxy en $C_1$-$C_4$, chacun étant éventuellement substitué par 1 à 5 atomes d'halogène; un halogène, un groupe cyano, alcynyle en $C_1$-$C_3$,alcényle en $C_1$-$C_3$, nitro, un groupe $S(O_n)R_7$, dans lequel n = 0, 1 ou 2, et $R_7$ est un groupe alkyle en $C_1$-$C_4$, éventuellement substitué par 1-5 halogènes, un groupe $NR_8R_9$, dans lequel $R_8$ et $R_9$ sont choisis indépendamment parmi un hydrogène ou un groupe alkyle en $C_1$-$C_4$, un groupe $=X_2$, dans lequel $X_2$ = O, S ou $NR_{10}$, où $R_{10}$ est choisi parmi un hydrogène, un groupe alkyle en $C_1$-$C_4$, alcoxy en $C_1$-$C_4$ et $COR_{11}$ où $R_{11}$ est un groupe alkyle en $C_1$-$C_4$.

Avantageusement, $R_1$ est un groupe pyridine, pyrrole, pyrazole, imidazole, thiazole, oxazole, isoxazole, isothiazole, triazole, pipéridine, morpholine, tétrahydropyranne, éventuellement substitué.

Avantageusement, les substituants du noyau $R_1$ sont des groupes alkyle en $C_1$-$C_4$, halogéno ou alkyle en $C_1$-$C_4$ substitué par 1-5 halogènes. De préférence, les substituants sont des groupes méthyle, éthyle, chloro, fluoro ou trifluorométhyle.

Lorsque Q est un système cyclique, parmi les substituants convenables, on peut citer les groupes hydrocarbyle en $C_1$-$C_6$, alcoxy en $C_1$-$C_6$ ou méthylènedioxy, chacun étant éventuellement substitué par un à cinq atomes d'halogène, ou groupes cyano ou nitro, ou le substituant est un groupe $S(O)_nR_7$, dans lequel n est égal à 1, 1 ou 2 et $R_7$ est un groupe en $C_1$-$C_6$ éventuellement substitué par un ou plusieurs halogènes ou $R_7$ est un groupe amino éventuellement substitué par un ou deux groupes alkyle en $C_1$-$C_6$, ou le substituant est un groupe $NR_8R_9$, dans lequel $R_8$ et $R_9$ sont choisis indépendamment parmi un hydrogène, ou un groupe alkyle en $C_1$-$C_6$ ou $COR_{12}$, dans lequel $R_{12}$ est un groupe alkyle en $C_1$-$C_6$, ou un groupe $COR_{12}$, dans lequel $R_{12}$ est un groupe alcoxy en $C_1$-$C_6$.

Avantageusement, Q est un système cyclique contenant de 5 à 10 atomes contenant un maximum de trois hétéroatomes choisis parmi N, O ou S, et les atomes de carbone restants sont éventuellement substitués, comme ci-dessus.

Avantageusement, Q est un groupe phényle, pyridyle, naphtyle, ou un système bicyclique à noyau condensés contenant un maximum de 3 hétéroatomes choisis parmi N, O ou S, chacun étant éventuellement substitué, comme ci-dessus.

De préférence, Q est un noyau aromatique monocyclique ou un système bicyclique à noyaux condensés dont au moins un noyau est aromatique et contient 0 ou 1 atome d'azote ou 0 ou 1 atome de soufre.

Lorsque Q est un noyau aromatique monocyclique, il s'agit avantageusement d'un groupe phényle, pyridyle, thiényle et, de préférence, phényle. Lorsque Q est un système bicyclique à noyaux, il s'agit de préférence d'un groupe naphtyle, quinoléinyle, tétrahydronaphtyle ou indanyle.

Le système cyclique Q contient normalement jusqu'à trois substituants et est avantageusement non substitué ou substitué par un, deux ou trois substituants tels que des groupes halogéno ou halogénoalkyle en $C_1$-$C_5$, comme un groupe trifluorométhyle. La substitution du système cyclique Q dépend de la nature de ce système cyclique, mais se fait de préférence en position 3, 4 ou 5 lorsque Q est un noyau à 6 chaînons. Avantageusement, $R_2$, $R_3$, $R_4$ et $R_5$ sont choisis parmi un hydrogène, un groupe méthyle ou un fluor. Avantageusement, la stéréochimie des doubles liaisons est (E). Avantageusement, lorsque $R_3$ ou $R_5$ est un fluor, la stéréochimie de la double liaison à laquelle est fixé $R_3$ ou $R_5$ est (Z).

De préférence, $R_2$ est un hydrogène, $R_3$ est un hydrogène ou un fluor, $R_5$ est un hydrogène ou un fluor et $R_4$ est un hydrogène ou un groupe alkyle en $C_1$-$C_4$, tout particulièrement méthyle.

De préférence, $Q_1$ est un groupe 1,2-cyclopropyle ou un groupe $(CH_2)m$.

De préférence, lorsque $Q_1$ est un noyau 1,2-cyclopropyle, b = 0, a = 0.

De préférence, la configuration stéréochimique du groupe cyclopropyle dans la chaîne est telle que les groupes Q et la chaîne carbonée latérale sont fixés au noyaux de manière à avoir une géométrie trans. De préférence, la position 3 du noyau cyclopropyle n'est pas substituée. Parmi les substituants qui conviennent sur les positions 1 et 2 du noyau cyclopropyle, on peut citer les groupes fluoro, chloro, méthyle ou trifluorométhyle. De préférence, la position 2 est non substituée et la position 1 est non substituée ou substituée par un fluor ou un chlore.

Lorsque $Q_1$ est $(CH_2)_m$ et que b = 1, la valeur de m est de préférence égale à 6 lorsque a = 1 et de préférence égale à 7 lorsque a = 0.

Un groupe de composés de formule (I) qui convient est celui de formule (II)

$$Q_aQ_{1a}CR_{2a} = CR_{3a}CR_{4a} = CR_{5a}C(=X_{1a})\,NHR_{1a} \qquad (II)$$

ou un de ses sels, formule dans laquelle $Q_a$ est un groupe phényle ou pyridyle éventuellement substitué, ou un système bicyclique à noyaux condensés éventuellement substitué, dont au moins un noyau est aromatique et qui contient 0 ou 1 atome d'azote ou 0 ou 1 atome de soufre.

$Q_{1a}$ est un noyau 1,2-cyclopropyle éventuellement substitué par un ou plusieurs groupes choisis parmi un groupe alkyle en $C_1$-$C_3$, halogéno ou halogénoalkyle en $C_1$-$C_3$; $R_{2a}$, $R_{3a}$, $R_{4a}$ et $R_{5a}$ sont identiques ou différents, l'un au moins étant un hydrogène et les autres étant choisis indépendamment parmi un hydrogène, un halogène, ou un groupe alkyle en $C_1$-$C_4$ ou halogénoalkyle en $C_1$-$C_4$; $X_{1a}$ est un oxygène ou un soufre; $R_{1a}$ est tel que défini ci-dessus.

Lorsque $Q_a$ contient un système aromatique, comme substituants convenables, on peut citer un ou plusieurs groupes choisis parmi les groupes halogéno, cyano, nitro, alkyle en $C_1$-$C_6$, alcoxy en $C_1$-$C_6$ et méthylènedioxy, chacun étant éventuellement substitué par un ou plusieurs halogènes, ou le substituant est un groupe $S(O)_nR_{7a}$, dans lequel n est égal à 0, 1 ou 2 et $R_{7a}$ est un groupe en $C_1$-$C_6$ éventuellement substitué par un halogène.

De préférence, $Q_a$ est un groupe phényle ou naphtyle ou pyridyle.

Avantageusement, $R_{2a}$, $R_{3a}$, $R_{4a}$ et $R_{5a}$ sont choisis parmi un hydrogène, un groupe méthyle et un fluor.

$R_{1a}$ est tel que défini ci-dessus.

Le groupe préféré de composés de formule (II) englobe ceux de formule (III)

$Q_aQ_{1a}CH=CHCR_{4a}=CHCONHR_{1a}$ (III)

dans laquelle $Q_a$, $Q_{1a}$, $R_{4a}$ et $R_{1a}$ sont tels que définis ci-dessus.

Un groupe préféré de composés de la présente invention englobe ceux de formule (IV)

$Q\ Q_1CH=CR_3CR_4=CR_5CONHR_1$ (IV)

dans laquelle Q, $Q_1$, $R_4$ et $R_1$ sont tels que définis ci-dessus.

Les composés préférés de formule (IV) englobent ceux dans lesquels Q est un groupe phényle substitué, $Q_1$ est un noyau 1,2-cyclopropyle trans, dans lequel la position 2 du noyau cyclopropyle est non substituée ou substituée par un groupe fluoro ou chloro, $R_4$ est un groupe méthyle ou un hydrogène, $R_3$ et $R_5$ sont des atomes d'hydrogène ou de fluor et $R_1$.

Le terme halogéno signifie fluoro, chloro, bromo et iodo.

Le terme hydrocarbyle signifie alkyle, alcényle, alcynyle, arylalkyle, y compris un groupe alkyle ou alcényle cyclique, éventuellement substitué par un groupe alkyle, alcényle ou alcynyle; et alkyle ou alcényle substitué par des groupes alkyle et alcényle cycliques, et phényle.

Les sels des composés de la présente invention sont normalement des sels d'addition avec un acide. Ces sels peuvent être formés à partir d'acides minéraux ou organiques ou cycloalkyliques. Parmi les sels que l'on préfère, on peut citer ceux formés à partir des acides chlorhydrique, bromhydrique, sulfurique, citrique, nitrique, tartrique, phosphorique, lactique, benzoïque, glutamique, aspartique, pyruvique, acétique, succinique, fumarique, maléique, oxaloacétique, hydroxynaphtoïque, iséthionique, stéarique, méthanesulfonique, éthanesulfonique, benzènesulfonique, toluène-p-sulfonique, lactobionique, glucuronique, thiocyanique, propionique, embonique, naphténoïque et perchlorique.

Les composés de formule (I) peuvent exister sous un certain nombre de formes stéréoisomères. La présente invention englobe à la fois les isomères géométriques et les stéréoisomères, et leurs mélanges. La présente invention englobe aussi les composés de formule (I) contenant des radioisotopes, en particulier ceux dans lequels un à trois atomes d'hydrogène sont remplacés par du tritium ou un ou plusieurs atomes de carbone sont remplacés par $^{14}C$.

Sous un autre aspect, la présente invention fournit un procédé de préparation d'un composé de formule (I), telle que définie ci-dessus, qui comprend

a) lorsque $X_1$ est un oxygène, la réaction de l'acide ou dérivé d'acide $Q(CH_2)_a(O)_bQ_1CR_2=CR_3CR_4=CR_5C(=X)Z_1$ correspondant avec une amine $H_2NR_1$, où Q, a, b, $Q_1$, $R_2$, $R_3$, $R_4$, $R_5$ et $R_1$ sont tels que définis ci-dessus et X est un oxygène et $Z_1$ est un groupe hydroxy, alcoxy en $C_1$-$C_6$, halogéno ou un ester phosphoroimidate (-P(O)(O-aryl)NH-aryle, où le groupe aryle est un groupe aryle en $C_6$-$C_{10}$).

b) la formation du groupment $CR_2CR_3CR_4=CR_5R_2=CR_3R_5C(=X_1)NHR_1$ par une réaction de type Wittig, et éventuellement, par la suite, la transformation d'un composé de formule (I) en un autre composé de formule (I) par des méthodes bien connues de l'homme de métier.

Le <u>procédé</u> (<u>a</u>) est normalement réalisé à une température non extrême, par exemple entre -25 et 150°C, dans un solvant aprotique anhydre, comme l'éther, le dichlorométhane, le toluène ou le benzène. Les conditions précises dépendront de la nature du groupe $Z_1$, par exemple, lorsque $Z_1$ est un groupe alcoxy, la réaction est avantageusement réalisée à une température élevée, à savoir de 50 à 125°C, et avantageusement au reflux, de préférence en présence d'un composé trialkylaluminium, tel que triméthylaluminium, qui forme un complexe avec l'amine $H_2NR_1$. Lorsque $Z_1$ est un halogène ou un phosphoroimidate, la réaction est avantageusement réalisée à une température de -20°C à 30°C, et de préférence en présence d'une amine tertiaire, comme la triéthylamine ou la pyridine.

Si le dérivé acide est un halogénure d'acide, par exemple le chlorure d'acide, il peut alors être formé à partir de l'acide correspondant par réaction avec un réactif convenable, comme le chlorure d'oxalyle ou le chlorure de thionyle. Lorsque $Z_1$ est un groupe phosphoroimidate, il est alors avantageusement formé à partir de (PhO)P(O)NHPhCl, dans lequel Ph est un groupe phényle. L'acide, ou la fonction acide dans le composé $Q(CH_2)_a(O)_bQ_1CR_2=CR_3CR_4=CR_5COZ_1$, peut être préparé par hydrolyse de l'ester correspondant.

Les esters peuvent être préparés par un certain nombre d'autres voies, par exemple

(i) Une réaction classique de Wittig ou de Wadsworth-Emmons, avec, par exemple, un aldéhyde et un triphénylphosphorane d'éthoxycarbonyl-méthylène, ou un anion de triéthylphosphono-crotonate ou de 3-méthyl-triéthylphosphono-crotonate.Cette dernière réaction peut conduire à un mélange d'isomères, par exemple un mélange de diénoates substitués (Z) et (E); un tel mélange peut être mis à réagir comme ci-dessus, et le mélange résultant d'amides séparé par chromatographie ou par d'autres techniques convenables. Le réactif de type Wittig peut être produit, par exemple, par la voie suivante, ou une variante de celle-ci

(1) (3)

$(CH_3)2C{=}CHCO_2Et \;\text{->}\; Z_2CH_2C(CH_3){=}CHCO_2Et \;\text{->}$

(2)

Wittig/Wadsworth-Emmons reagent

dans laquelle $Z_2$ = $(aryl)_3P$, $(aryl)_2P(O)$ ou (alcoxy en $C_1$-$C_4$)$_2P(O)$, où le groupe aryle est de préférence un groupe phényle et le groupe alcoxy est de préférence un groupe éthoxy.

(1) N-bromosuccinimide

(2) par exemple $(EtO)_3P$ ou $(Ph)_3P$

(3) Cette réaction est normalement réalisée en présence d'une base, comme le lithium-diisopropyl amide, le butyl-lithium, un alcoxyde de sodium ou l'hydrure de sodium.

(ii) Par transposition et élimination de $HS(\rightarrow O)Z^3$ d'un composé de formule:

```
                                              S(->O)Z3
                                             /
Q(CH2)a(O)bQ1CHR2CHR3CR4 = C
                                             \
                                              COZ1
```

dans laquelle Q, $Q_1$, $R_2$, $R_3$ et $R_4$ sont tels que définis ci-dessus, $Z_3$ est n'importe quel groupe convenable, par exemple phényle, phényle substitué tel que 4-chlorophé-nyle, ou alkyle en $C_1$-$C_4$, par exemple méthyle, $Z_1$ est tel que défini ci-dessus et préférablement un groupe alkoxy en $C_1$-$C_4$, par exemple méthoxy ou éthoxy.

Le composé ci-dessus peut être obtenu par réaction d'un composé $Q(CH_2)_a(O)_bQ_1CHR_2CHR_3CR_4O$ avec un composé $Z_3$ $S(O)CH_2CO_2Z_1$.

(iii) Par élimination de $HOZ_5$ d'un composé $Q(CH_2)_a(O)_bQ_1CHR_2CR_3(OZ_5)CR_4=CR_5CO_2Z_1$, dans lequel Q, a, b, $Q_1$, $R_2$, $R_3$, $R_4$, $R_5$ et $Z_1$ sont tels que définis ci-dessus, et $Z_5$ représente un hydrogène ou un groupe acyle en $C_1$-$C_4$ tel que le groupe acétyle. La réaction est réalisée de préférence dans un solvant aromatique, avantageusement en présence d'un catalyseur au molybdène et d'une base, comme le bistriméthylsilylacétamide.

On peut obtenir le composé ci-dessus en faisant réagir un aldéhyde convenable avec un composé sulfényle convenable, puis en effectuant une acylation.

(iv) Par réaction d'un composé de formule $Q(CH_2)_a(O)_bQ_1CR_2=CR_3C(=O)R_4$ avec un composé de formule $Me_3SiCHR_5CO_2Z_1$, formules dans lesquelles Q, a, b, $R_2$ à $R_5$, $Q_1$ et $Z_1$ sont tels que définis ci-dessus.

On peut mettre en oeuvre ce procédé dans un solvant anhydre, par exemple du tétrahydrofuranne en l'absence d'oxygène, en présence d'une base, par exemple le lithium-cyclohexylisopropylamide.

(v) Par réaction d'un composé de formule $Q(CH_2)_a(O)_bQ_1CR_2=CR_3C(OZ_6)=CR_5CO_2Z_1$, avec un composé de formule $R^4M^1$, formules dans lesquelles Q, a, b, $Q_1$, $R_2$, $R_3$, $R_4$, $R_5$ et $Z_1$ sont tels que définis ci-dessus, $Z_6$ est un groupe convenable, tel que dialkylphosphate ou trifluorométhanesulfonate, et $M_1$ est un métal tel que le cuivre (I) ou le cuivre (I) associé au lithium ou au magnésium.

Ce procédé peut être mis en oeuvre à une température faible dans un solvant éthéré anhydre, comme l'éther diéthylique, le sulfure de diméthyle ou le tétrahydrofuranne, en l'absence d'oxygène.

(vi) Par réaction d'un composé de formule $Q(CH_2)_a(O)_bQ_1CR_2=CR_3M_2$ avec un composé de formule Y $CR_4=CR_5CO_2Z_4$, formules dans lesquelles Q, a, b, $Q_1$, $R_2$, $R_3$, $R_4$, $R_5$ et $Z_1$ sont tels que définis ci-dessus, Y est un atome d'halogène ou d'étain, et $M_2$ est un groupe silyle ou métallique, tel que triméthylsilyle ou un groupe contenant du zirconium, de l'étain, de l'aluminium ou du zinc, par exemple un groupe chlorure de bis(cyclopentadiényl) zirconium. Ce procédé est normalement réalisé à une température non extrême, à savoir comprise entre 0 et 100°C, et avantageusement à la température ambiante, dans un solvant éthéré non aqueux, tel que le tétrahydrofuranne, en présence d'un catalyseur au palladium (0) (tel que le bis(triphénylphosphine)-palladium) et dans une atmosphère inerte d'azote ou d'argon.

(vii) Par élimination de $Z_3S(\text{->}O)H$ d'un composé de formule

$$Q(CH_2)_a(O)_bQ_1CR_2=CR_3CHR_4\underset{\displaystyle |\atop S(\text{->}O)Z_3}{CR_5}COZ_1$$

dans laquelle Q, a, b, $Q_1$, $R_2$, $R_3$, $R_4$, $R_5$, $Z_3$ et $Z_1$ sont tels que définis ci-dessus.

Le composé ci-dessus peut être obtenu par réaction d'un composé $QQ_1CHR_2CR_3=CHR_4$ avec $Z_3S(O)$-$CH_2CO_2Z_1$.

On peut mettre en oeuvre le procédé (b) en ayant un groupe aldéhyde ou cétone fixé ou bien à l'extrémité amide/thioamide, ou bien sur le fragment $QQ_1$ de formule (I), puis en faisant réagir ce composé avec l'ylide phosphoreux approprié.

Autrement dit

$Q(CH_2)_a(O)_bQ_1(CR_2=CR_3)COR_4$ + $Z_2CHR_5.C(=X)NHR_1$ ou $Q(CH_2)_a(O)_bQ_1COR_2$ + $Z_2CHR_3.CR_4=CR_5.C$-$(=X)NHR_1$ ou $Q(CH_2)_a(O)_bQ_1(CR_2=CR_3)CHR_5Z_2$ + $R_5CO.C(=X)NH.R_1$

où Q, a, b, $Q_1$, $R_2$, $R_3$, $R_4$, $R_5$, $R_1$, X et $Z_2$ sont tels que définis ci-dessus.

Le procédé (b) est mis en oeuvre dans un solvant inerte anhydre, par exemple un éther tel que le tétrahydrofuranne, ou un alcool tel que le méthanol, éventuellement en présence d'une base, et de préférence en l'absence d'oxygène, par exemple sous une atmosphère d'azote, à une température faible (-60°C à 20°C). L'ylide phosphoreux peut être obtenu à partir de son précurseur, comme on l'a décrit ci-dessus, par réaction avec une base telle que le lithium-diisopropylamide, le butyllithium, un alcoxyde de sodium, l'hydrure de sodium, le carbonate de potassium ou de sodium. Les composés de formule (I), dans laquelle X représente un atome de soufre, sont de préférence préparés par le procédé (b) lorsque $Z_2$ représente un groupe (alcoxy en $C_1$-$C_4$)$_2P=O$.

On prépare les précurseurs de type $Z_2CHR_3CR_4$-$CR_5(C=X_1)NHR$ selon le procédé suivant, ou une variante de celui-ci, lorsque $X_1$ = oxygène.

$$CH_3CR_4=CR_5CO_2H \xrightarrow[2)]{1)} BrCH_2CR_4=CR_5COCl$$

$$\xrightarrow{3)} BrCH_2CR_4=CR_5CONHR_1 \xrightarrow{4)} Z_2CH_2CR_5=CR_5CONHR_1$$

1) N-bromosuccinimide
2) chlorure de thionyle
3) $NH_2R_1$
4) $(EtO)_3P$, lorsque $Z_2$ = $(OEt)_2P(O)$-

Lorsque $X_1$ représente un atome de soufre, on prépare des précurseurs de type $Z_2CHR_5(C=S)NHR_1$ par réaction de l'anion dérivé de $Z_2$ $CH_2R_5$ avec $R_1NCS$.

Lorsque $X_1$ est un oxygène, on prépare les précurseurs de type Z $CHR_5(C=O)NHR_1$ par réaction de $Ph_3P$ ou de $P(OEt)_3$ avec $ClCHR_5(CO)NHR_1$.

On peut préparer les intermédiaires aldéhydes $Q(CH_2)_a(O)_bQ_1CR_2=O$ par hydrolyse acide d'un cétal, d'un énoléther ou d'un acétal dans un solvant tel qu'un système acétone-eau, ou par oxydation d'alcools appropriés à l'aide, par exemple, de chlorochromate de pyridinium, de dichromate de pyridinium ou de chlorure d'oxalyle/diméthylsulfoxyde, dans un solvant tel que le dichlorométhane. On peut aussi préparer les aldéhydes par réduction des nitriles appropriés avec un réactif tel que l'hydrure de diisobutylaluminium dans l'hexane Les intermédiaires de la présente invention forment un autre aspect de la présente invention et peuvent être préparés, le cas échéant, par des procédés standards autres que ceux décrits.

Les composés de formule (I) peuvent être utilisés pour lutter contre les nuisibles tels que les arthropodes, par exemple les insectes et les acariens nuisibles, et les helminthes, à savoir les nématodes. Ainsi, la présente invention fournit un procédé de lutte contre les arthropodes et/ou les helminthes, qui comprend l'administration à l'arthropode et/ou à l'helminthe, ou dans leur environnement, d'une quantité efficace contre les arthropodes d'un composé de formule (I). La présente invention fournit aussi un procédé pour lutter et/ou éradiquer les infestations par les arthropodes et/ou les helminthes d'animaux (notamment de l'homme) et/ou de végétaux (notamment d'arbres) et/ou de produits stockés, qui comprend l'administration à l'animal ou au lieu d'une quantité efficace d'un composé de formule (I). La présente invention fournit, en outre, les composés de formule (I) à utiliser en médecine humaine et vetérinaire, dans le contrôle de la santé publique et en agriculture pour lutter contre les nuisibles de type arthropode et/ou helminthe.

Les composés de formule (I) sont particulièrement intéressants dans la protection des cultures en champ, en fourrage, en plantation, en serre, en verger et en vigne, des arbres ornementaux et des arbres de plantations et de forêts, par exemple des céréales (telles que le maïs, le blé, le riz, le sorgho), du coton, du tabac, des légumes et des salades (comme les haricots, les choux, les cucurbitacés, les laitues, les oignons, les tomates et les poivrons), des cultures en champ (comme la pomme de terre, la betterave à sucre, l'arachide, le soja, la luzerne), des cannes à sucre, des prairies et du fourrage (comme le maïs, le sorgho, la luzerne), des plantations (telles que le thé, le café, le cacao, la banane, la palme à huile, la noix de coco, le caoutchouc, les épices), des vergers et des plantations d'arbres (comme les fruits à noyau et les fruits à pépins, les agrumes, les kiwis, les avocats, les mangues, les olives et les noix), les vignes, les plantes ornementales, les fleurs et les buissons sous serre et dans les jardins et les parcs, les arbres forestiers (à feuilles caduques ou à feuilles persistantes) dans les forêts, les plantations et les pépinières.

Ils sont aussi intéressants dans la protection du bois (sur pied, tombé, transformé, stocké ou de construction) contre l'attaque des tenthrèdes (par exemple Urocerus) ou des coléoptères (par exemple les scolytidés, les platypodes, les lyctes, les bostrychidés, les cérambycidés, les anobiidés).

Ils présentent des applications dans la protection des produits stockés, comme les grains, les fruits, les noix, les épices et le tabac, qu'ils soient entiers, broyés ou mélangés dans des produits, contre l'attaque des mites, des coléoptères et des acariens. Sont aussi protégés les produits animaux stockés comme les peaux, les pelages, la laine et les plumes, sous forme naturelle ou transformée (par exemple de tapis ou de textiles) de l'attaque des mites et des coléoptères; de même que la viande stockée et le poisson stocké contre l'attaque des coléoptères, des acariens et des mouches.

Les composés de formule générale (I) sont particulièrement intéressants dans la lutte contre les arthropodes ou les helminthes, qui sont dangereux pour l'homme et les animaux domestiques, ou répandent ou jouent le rôle de vecteurs de maladies chez l'homme et les animaux domestiques, par exemple ceux que l'on a cités ci-dessus, et plus particulièrement dans la lutte contre les tiques, les acariens, les poux, les puces, les moucherons et les mouches qui piquent, les mouches qui gênent et les mouches à myiase.

Les composés de formule (I) peuvent être utilisés pour ces besoins par application des composés eux-mêmes, ou sous une forme diluée, d'une façon connue, par exemple par trempage, vaporisation, brouillard, laque, mousse, poussière, poudre, suspension aqueuse, pâte, gel, crème, shampooing, graisse, solide combustible, tampon de vaporisation, serpentin combustible, appât, complément alimentaire, poudre mouillable, granulé, aérosol, concentré émulsionnable, suspensions huileuses, solutions huileuses, bombe pressurisée, article imprégné, formulation à verser ou d'autres formulations standards bien connues de l'homme de métier. Les concentrés pour trempage ne sont pas appliqués seuls, mais dilués avec de l'eau, et les animaux sont immergés dans un bain de trempage contenant le produit de trempage. Les vaporisations peuvent être appliquées à la main, ou au moyen d'un chemin ou d'un arc de projection. L'animal, le sol, la plante ou la surface à traiter peut être saturé de la vaporisation grâce à une application à volume élevé, ou enduit superficiellement par la vaporisation au moyen d'une application légère ou de volume ultra-faible. On peut appliquer des suspensions aqueuses de la même manière que des vaporisations ou des trempages. Les poussières doivent être distribuées au moyen d'un applicateur de poudre ou, dans le cas d'animaux, incorporées dans des sacs perforés fixés à des arbres ou des barres de frottement. Des pâtes, des shampooings et des graisses peuvent être appliqués à la main ou distribués sur la surface

d'un matériau inerte, comme celui contre lequel les animaux se frottent, et qui transfère le produit sur leur peau. Les formulations à verser sont distribuées en unités de liquide de faible volume sur le dos des animaux, de manière à ce que la totalité ou la plupart du liquide soit retenue sur les animaux.

Les composés de formule (I) peuvent être préparés en formulations prêtes à l'emploi sur les animaux, les plantes ou sur la surface, ou sous forme de formulations nécessitant une dilution avant l'application, mais les deux types de formulations comprennent un composé de formule (I) en mélange intime avec un ou plusieurs véhicules ou diluants. Les véhicules peuvent être liquides, solides ou gazeux, ou comprendre des mélanges de telles substances, et le composé de formule (I) peut être présent en une concentration de 0,025 à 99% en poids/vol., suivant que la formulation nécessite ou non une dilution plus poussée.

Les poussières, les poudres et les granulés, et d'autres formulations solides, comprennent le composé de formule (I) en mélange intime avec un véhicule solide inerte, pulvérulent, par exemple des argiles convenables, du kaolin, de la bentonite, de l'attapulgite, du noir de carbone adsorbant, du talc, du mica, de la craie, du gypse, du phosphate tricalcique, du liêge pulvérulent, du silicate de magnésium, des véhicules végétaux, de l'amidon et de la diatomite. On prépare en général de telles formulations solides par imprégnation des diluants solides par des solutions du composé de formule (I) dans des solvants volatils, évaporation des solvants et, le cas échéant, broyage des produits pour obtenir des poudres et, le cas échéant, granulation, compactage ou encapsulation des produits.

Les vaporisations d'un composé de formule (I) peuvent comprendre une solution dans un solvant organique (par exemple ceux énumérés ci-dessus) ou une émulsion dans de l'eau (lavage par trempage ou vaporisation), préparée sur le champ à partir d'un concentré émulsionnable (connu autrement sous le nom d'huile miscible à l'eau), que l'on peut utiliser pour le trempage. Le concentré comprend de préférence un mélange de l'ingrédient actif, avec ou sans solvant organique, et d'un ou de plusieurs émulsifiants. Des solvants peuvent être présents en quantités très variables, mais de préférence en une quantité de 0 à 90% en poids/volume de la composition, et peuvent être choisis parmi le kérosène, les cétones, les alcools, le xylène, le naphta aromatique, et d'autres solvants connus dans la technique de la formulation. La concentration des émulsifiants peut avoir une valeur très variable, mais est de préférence dans la plage de 5 à 25% en poids/volume, et les émulsifiants sont avantageusement des agents tensioactifs non ioniques, notamment des esters polyalcoxylés d'alkylphénols et des dérivés polyéthoxylés d'anhydride d'hexitol et des agents tensioactifs anioniques, en particulier le laurylsulfate de Na, les éthersulfates d'alcools gras, les sels de Na et de Ca d'alkylarylsulfonates et d'alkylsulfosuccinates. Les émulsifiants cationiques englobent le chlorure de benzalconium et les éthylsulfates d'ammonium quaternaire.

Les émulsifiants amphotères englobent l'imidazoline oléique carboxyméthylée et les alkyldiméthylbétaïnes.

Les mèches de vaporisation comprennent normalement un mélange de coton et de cellulose comprimé pour donner une plaque dont les dimensions sont d'environ 35 x 22 x 3 mm, traitée avec jusqu'à 0,3 ml de concentré comprenant l'ingrédient actif dans un solvant organique et éventuellement un antioxydant, un colorant et un parfum. On vaporise l'insecticide en utilisant une source de chaleur, comme un dispositif de chauffage du tampon fonctionnant à l'électricité.

Les solides combustibles comprennent normalement de la poudre de bois et un liant mélangés avec l'ingrédient actif et mis sous forme de bandes moulées (habituellement en serpentins). Un colorant et un fongicide peuvent aussi être ajoutés. Les poudres mouillables comprennent un véhicule solide inerte, un ou plusieurs agents tensioactifs, et éventuellement des stabilisants et/ou des anti-oxydants.

Les concentrés émulsionnables comprennent des agents émulsifiants et souvent un solvant organique, comme le kérosène, les cétones, les alcools, les xylènes, les naphtes aromatiques et d'autres solvants connus dans la technique.

Les poudres mouillables et les concentrés émulsionnables contiennent normalement de 5 à 95% en poids de l'ingrédient actif et sont dilués, par exemple avec de l'eau, avant l'emploi.

Les laques comprennent une solution de l'ingrédient actif dans un solvant organique, avec une résine et éventuellement un plastifiant.

On peut préparer des produits de lavage par trempage, non seulement à partir de concentrés émulsionnables, mais aussi à partir de poudres mouillables, de produits de trempage à base de savon et de suspensions aqueuses comprenant un composé de formule (I) en mélange intime avec un agent dispersant et un ou plusieurs agents tensioactifs.

Les suspensions aqueuses d'un composé de formule (I) peuvent comprendre une suspension dans l'eau ainsi que des agents de mise en suspension, des stabilisants ou autres. Les suspensions ou les solutions peuvent être appliquées telles quelles ou sous une forme diluée de façon connue.

On peut préparer des graisses (ou pommades) à partir d'huiles végétales, d'esters synthétiques d'acides gras ou de lanoline, avec une base inerte comme la paraffine molle. Un composé de formule (I)

est de préférence distribué uniformément dans le mélange, en solution ou en suspension. Les graisses peuvent aussi être faites à partir de concentrés émulsionnables par dilution avec une base pour pommade.

Les pâtes et les shampooings sont aussi des préparations semi-solides dans lesquelles un composé de formule (I) peut être présent en dispersion uniforme dans une base convenable comme de la paraffine molle ou liquide, ou préparé sur une base non grasse avec de la glycérine, un mucilage ou un savon convenable. Comme les graisses, les shampooings et les pâtes sont normalement appliqués sans autre dilution, ils doivent contenir le pourcentage approprié du composé de formule (I) requis pour le traitement.

On peut préparer des vaporisations en aérosol sous forme d'une simple solution de l'ingrédient actif dans le propulseur aérosol et le co-solvant, comme des alcanes halogénés et les solvants mentionnés ci-dessus, respectivement.

On peut fabriquer les formulations à verser en solution ou en suspension d'un composé de formule (I) dans un milieu liquide. Un hôte aviaire ou mammifère peut aussi être protégé contre l'infestation d'ectoparasites acariens grâce au port d'un article en plastique moulé de forme convenable, imprégné d'un composé de formule (I). Comme articles de ce type, on peut citer les coliers imprégnés, les étiquettes, les bandes, les feuilles et les rubans fixés de façon adéquate aux parties du corps appropriées. Avantageusement, la matière plastique est un poly(chlorure de vinyle) (PVC).

La concentration du composé de formule (I) à appliquer sur un animal, dans des locaux ou sur des zones a l'extérieur variera suivant le composé choisi, l'intervalle entre les traitements, la nature de la formulation et l'infestation probable, mais, en général, il faut que 0,001 à 20,0% en poids/vol et de préférence 0,01 à 10%, du composé soit présent dans la formulation appliquée. La quantité de composé déposée sur un animal variera suivant la méthode d'application, la taille de l'animal, la concentration du composé dans la formulation appliquée, le facteur de dilution de la formulation et la nature de la formulation, mais se situe en général dans la gamme de 0,0001% à 0,5% en poids/poids, sauf pour les formulations non diluées, comme les formulations à verser, qui sont en général déposées à une concentration située dans la gamme de 0,1 à 20,0%, et de préférence de 0,1 à 10%. La quantité de composé à appliquer à des produits stockés se situe en général dans la gamme de 0,1 à 20 ppm. On peut appliquer des vaporisations de l'espace pour obtenir une concentration moyenne initiale de 0,001 à 1 mg de composé de formule (I) par mètre cube d'espace traité.

Les composés de formule (I) sont aussi utiles dans la protection et le traitement des espèces végétales, auquel cas on applique une quantité efficace insecticide, acaricide ou nématocide de l'ingrédient actif. Le taux d'application variera suivant le composé choisi, la nature de la formulation, le mode d'application, l'espèce végétale, la densité de plantation et l'infestation probable, et selon d'autres facteurs de ce genre, mais, en général, un taux d'utilisation convenable pour les récoltes agricoles se situe dans la gamme de 0,001 à 3 kg/ha, et de préférence de 0,01 à 1 kg/ha. Les formulations typiques à usage agricole contiennent entre 0,0001% et 50% d'un composé de formule (I) et, avantageusement, entre 0,1 et 15% en poids d'un composé de formule (I).

Les poussières, les graisses, les pâtes et les formulations en aérosol sont habituellement appliquées d'une façon aléatoire, comme décrit ci-dessus, et on peut employer des concentrations de 0,001 à 20% en poids/vol. d'un composé de formule (I) dans la formulation appliquée.

On a constaté que les composés de formule (I) avaient une activité contre la mouche domestique (Musca domestica). De plus, certains composés de formule (I) ont une activité contre d'autres nuisibles arthropodes, notamment Myzus persicae, Tetranychus urticae, Plutella xylostella, Culex spp., Tribolium castaneum, Sitophilus granarius, Periplaneta americana et Blatella germanica. Les composés de formule (I) sont ainsi utiles pour lutter contre les arthropodes, par exemple les insectes et les acariens, dans n'importe quel environnement dans lequel ils sont nuisibles, par exemple en agriculture, en élevage, dans le contrôle de la santé publique et dans les situations domestiques.

Parmi les membres des insectes nuisibles, on peut citer l'ordre des coléoptères (par exemple Anobium, Ceutorhynchus, Rhynchophorus, Cosmoppolites, Lissorhoptrus, Meligethes, Hypothenemus, Hylesinus, Acalymma, Lema, Psylliodes, Leptinotarsa, Gonocephalum, Agriotes, Dermoleoida, Heteronychus, Phaedon, Tribolium, Sitophilus, Diabrotica, Anthonomus ou Anthrenus spp.), l'ordre des lépidoptères (par exemple Ephestia, Mamestra, Earias, Pectinophora, Ostrinia, Trichoplusia, Pieris, Laphygma, Agrotis, Amathes, Wiseana, TryDorysa, Diatraea, Sporganothis, Cydia, Archips, Plutella, Chilo, Heliothis, Spodoptera ou Tineola spp.), l'ordre des diptères (par exemple Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza et Melophaaus spp.), l'ordre des phtiraptères (Malophaga, par exemple Damalina spp. et Anoplura, par exemple Linognathus et

Haematopinus spp.), l'ordre des hémiptères (par exemple Aphis, Bemisia, Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleurodes, Triatoma, Psylla, Mysus, Megoura, Phylloxera, Adelyes, Niloparvata, Nephrotetix ou Cimex spp.), l'ordre des orthoptères (par exemple Locusta, Gryllus, Schistocerca ou Acheta spp.), l'ordre des dictyoptères (par exemple Blatella, Periplaneta ou Blatta spp.), l'ordre des hyménoptères (par exemple Athalia, Cephus, Atta, Solenopsis ou Monomorium spp.), l'ordre des isoptères (par exemple Odontotermes et Reticulitermes spp.), l'ordre des siphonaptères (par exemple Ctenocephalides ou Pulex spp.), l'ordre des thysanoures (par exemple Lepisma spp.), l'ordre des dermaptères (par exemple Forficula spp.), l'ordre des psocoptères (par exemple Peripsocus spp.) et l'ordre des thysanoptères (par exemple Thrips tabaci).

Les acariens nuisibles englobent les tiques, par exemple les membres des genres Boophilus, Ornithodorus, Rhipicephalus, Ixodes, Haemaphysalis, Dermacentor et Anocentor, et les acariens et les gales tels que Acarus, Tetranychus, Psaroptes, Notaednes, Sarcaptes, Psorergates, Chorioptes, Eutrombicula, Demodex, Panonychus, Bryobia, Eriophyes, Blaniulus, Polyphagotarsonemus, Scutiaerella et Oniscus spp.

Les nématodes qui attaquent les plantes et les arbres importants en agriculture, en sylviculture, en horticulture, ou bien directement, ou bien en répandant les maladies bactériennes, virales, mycoplasmiques ou fongiques des plantes, englobent les nématodes des noeuds des racines, tels que Meloidogyne spp. (par exemple M. incoanita); les nématodes des kystes, tels que Globodera spp. (par exemple G. rostochiensis); Heterodera spp. (par exemple hydrogen. avenae); Rhadopholus spp. (par exemple R. similis); les nématodes des lésions, tels que Pratylenchus spp. (par exemple P. pratensis); Belonolaimus spp. (par exemple B. gracilis); Tylenchulus spp. (par exemple T. semipenetrans); Rotylenchulus spp. (par exemple R. reniformis); Rotylenchus spp. (par exemple R. robustus); Helicotylenchus spp. (par exemple hydrogen. multicinctus); Kemicycliophora spp. (par exemple hydrogen. pracilis); Criconemoides spp. (par exemple C. similis); Trichodorus spp. (par exemple T. primitivus); les nématodes à dague, tels que Xiphinema spp. (par exemple X. diversicaudatum), Longidorus spp. (par exemple L. elongatus); Hoplolaimus spp. (par exemple hydrogen.coronatus); Aphelenchoides spp. (par exemple A ritzema-bosi, A. besseyi); les anguillules des tiges et des bulbes, tels que Ditylenchus spp. (par exemple D. dipsaci).

Les composés de l'invention peuvent être associés à un ou plusieurs autres ingrédients actifs en tant que pesticides (par exemple pyréthroïdes, carbamates et organophosphates) et/ou avec des agents attirants, repoussants, bactéricides, fongicides, nématocides, antihelmintiques et analogues. En outre, on a constaté que l'activité des composés de l'invention peut être améliorée si l'on ajoute un agent synergique ou agent un potentialisateur, par exemple un composé de la classe des agents synergiques inhibiteurs d'oxydase, comme le butylate de pipéronyle, ou le 2-propynylphénylphosphonate de propyle; un second composé de l'invention; ou un composé pesticide de la famille des pyréthroïdes. Lorsqu'un agent synergique inhibiteur d'oxydase est présent dans une formule de l'invention, le rapport de l'agent synergique au composé de formule (I) se situe dans la gamme de 25:1-1:25, par exemple d'environ 10:1.

Comme stabilisants servant à empêcher une décomposition chimique éventuelle des composés de l'invention, on peut citer, par exemple, les antioxydants (comme les tocophérols, le butylhydroxy anisole et le butylhydroxytoluène) et les piégeurs (comme l'épichlorhydrine) et les bases organiques ou minérales, par exemple les trialkylamines, comme la triéthylamine, qui peut jouer le rôle de stabilisant basique et de piégeur.

Applications industrielles

Les composés de la présente invention prés- entent des propriétés pesticides accrues et/ou une stabilité à la lumière et/ou une toxicité réduite pour les mammifères.

Les exemples suivants illustrent, sans la limiter, les aspects préférés de l'invention.

Formulations

1. Concentré émulsionnable

| | |
|---|---|
| Composé de formule (I) | 10,00 |
| Ethoxylat d'alkylphénol* | 7,50 |
| Alkylarylsulfonate* | 2,50 |
| Solvant aromatique en $C_8$-$C_{13}$ | 80,00 |
| | 100,00 |

* Tensioactif

2. Concentré émulsionnable

| | |
|---|---|
| Composé de formule (I) | 10,00 |
| Ethoxylat d'alkylphénol* | 2,50 |
| Alkylarylsulfonate* | 2,50 |
| Solvant cétonique | 64,00 |
| Solvant aromatique en $C_8$-$C_{13}$ | 18,00 |
| Antioxydant | 3,00 |
| | 100,00 |

* Tensioactif

3. Poudre mouillable

| | |
|---|---|
| Composé de formule (I) | 5,00 |
| Solvant aromatique en $C_8$-$C_{13}$ | 7,00 |
| Solvant aromatique en $C_{18}$ | 28,00 |
| Argile à porcelaine | 10,00 |
| Alkylarylsulfonate* | 1,00 |
| Acide naphtalènesulfonique* | 3,00 |
| Diatomite | 46,00 |
| | 100,00 |

* Tensioactif

4. Poussière

| | |
|---|---|
| Composé de formule (I) | 0,50 |
| Talc | 99,50 |
| | 100,00 |

5. Appât

| | |
|---|---|
| Composé de formule (I) | 0,5 |
| Sucre | 79,5 |
| Cire de paraffine | 20,00 |
| | 100,00 |

6. Concentré d'émulsion

| | |
|---|---|
| Composé de formule (I) | 5,00 |
| Solvant aromatique en $C_8$-$C_{13}$ | 32,00 |
| Alcool cétylique | 3,00 |
| Monooléate de polyoxyéthylèneglycérol* | 0,75 |
| Esters de sorbitanne polyéthoxylés* | 0,25 |
| Solution de silicone | 0,1 |
| Eau | 58,9 |
| | 100,00 |

* Tensioactif

7. Concentré de suspension

| | |
|---|---|
| Composé de formule (I) | 10,00 |
| Ethoxylat d'alkylaryle* | 3,00 |
| Solution de silicone | 0,1 |
| Alcanediol | 5,0 |
| Silice fumée | 0,50 |
| Gomme xanthane | 0,20 |
| Eau | 80,0 |
| Agent tampon | 1,2 |
| | 100,00 |

* Tensioactif

8. Microémulsion

| | |
|---|---|
| Composé de formule (I) | 10,00 |
| Monooléate de polyoxyéthylèneglycérol* | 10 ,00 |
| Alcanediol | 4,00 |
| Eau | 76,00 |
| | 100,00 |

* Tensioactif

9. Granulés dispersables dans l'eau

| | |
|---|---|
| Composé de formule (I) | 70,00 |
| Polyvinylpyrrolidine | 2,50 |
| Ethoxylat d'alkylaryle | 1,25 |
| Alkylaryl sulfonate | 1,25 |
| Argile à porcelaine | 25,00 |
| | 100,00 |

10. Granulés

| | |
|---|---|
| Composé de formule (I) | 2,00 |
| Ethoxylat d'alkylaryle* | 5,00 |
| Alkylaryl sulfonate* | 3,00 |
| Solvant aromatique en $C_8$-$C_{13}$ | 20,00 |
| Granulés de kieselguhr | 70,00 |
| | 100,00 |

* Tensioactif

11. Aérosol (bombe pressurisée)

| | |
|---|---|
| Composé de formule (I) | 0,3 |
| Butylate de pipéronyle | 1,5 |
| Solvant hydrocarboné saturé en $C_8$-$C_{13}$ | 58,2 |
| Butane | 40,0 |
| | 100,00 |

12. Aérosol (bombe pressurisée)

| | |
|---|---|
| Composé de formule (I) | 0,3 |
| Solvant hydrocarboné saturé en $C_8$-$C_{13}$ | 10,0 |
| Monooléate de sorbitanne* | 1,0 |
| Eau | 40,0 |
| Butane | 48,7 |
| | 100,00 |

* Tensioactif

13. Aérosol (bombe pressurisée)

| | |
|---|---|
| Composé de formule (I) | 1,00 |
| $CO_2$ | 3,00 |
| Monooléate de glycérol polyéthoxylé* | 1,40 |
| Propanone | 38,00 |
| Eau | 56,60 |
| | 100,00 |

* Tensioactif

14. Lague

| | |
|---|---|
| Composé de formule (I) | 2,50 |
| Résine | 5,00 |
| Antioxydant | 0,50 |
| White spirit à forte teneur en aromatiques | 92,0 |
| | 100,00 |

15. Vaporisation (prête à l'emploi)

| | |
|---|---|
| Composé de formule (I) | 0,10 |
| Antioxydant | 0,10 |
| Kérosène inodore | 99,8 |
| | 100,00 |

16. Vaporisateur potentialisé (prêt à l'emploi)

| | |
|---|---|
| Composé de formule (I) | 0,10 |
| Butylate de pipéronyle | 0,50 |
| Antioxydant | 0,10 |
| Kérosène inodore | 99,30 |
| | 100,00 |

17. Microencapsulé

| | |
|---|---|
| Composé de formule (I) | 10,0 |
| Solvant aromatique en $C_8$-$C_{13}$ | 10,0 |
| Di-isocyanate aromatique | 4,5 |
| Ethoxylat d'alkylphénol* | 6,0 |
| Alkyldiamine* | 1,0 |
| Diéthylènetriamine | 1,0 |
| Acide chlorhydrique concentré | 2,2 |
| Gomme xanthane | 0,2 |
| Silice fumée | 0,5 |
| Eau | 64,6 |
| | 100,00 |

* Tensioactif
# réagit pour former les parois de polyurée de la microcapsule

L'antioxydant peut être n'importe lequel des composés suivants, individuellement ou en combinaison:
Hydroxytoluène butylé
Hydroxyanisole butylé
Vitamine C (acide ascorbique)

Les exemples suivants illustrent, sans la limiter, les aspects préférés de l'invention.

## PARTIE EXPERIMENTALE

### Méthodes et procédures de synthèse générales

Divers composés sont synthétisés et caractérisés selon les procédures expérimentales suivantes.

On obtient des spectres de RMN de $^1H$ sur un spectromètre Bruker AM-250 dans des solutions de deutérochloroforme avec du tétraméthylsilane comme étalon interne, exprimés en ppm à partir de TMS, nombre de protons, nombre de pics, constante de couplage J en Hz.

On peut aussi avantageusement surveiller le déroulement des réactions sur des tôles d'aluminium (40 x 80 mm) préenduites de couches de 0,25 mm de gel de silice, avec un indicateur fluorescent, développées dans un solvant ou un mélange de solvants approprié. La température est exprimée en degrés Celsius tout au long du texte.

L'éther diéthylique, l'hexane, l'éthanol, le méthanol, la triéthylamine, la pyridine, le sulfate de magnésium et l'hydroxyde de sodium s'obtiennent auprès de BDH. Le dichloro-méthane s'obtient auprès de Romil Chemicals et le diméthylformamide auprès de Rathburn Chemicals Ltd. La 2-aminopyridine, la 2-amino-3-méthylpyridine, le 2-aminothiazole, le 3-amino-5-méthyloxazole, le 2-amino-1,3,4-thiadiazole, la 4-aminopyrimidine, le 3-aminopyrazole, la 4-aminopipéridine, la 1-amino-2,6-diméthylpipéridine, la 4-aminomorpholine, la N-aminohexaméthylèneimine s'obtiennent auprès de Aldrich. Le 2-amino-4-trifluorométhyl-1,3-thiazole et le 2-amino-5-chloro-4-trifluorométhyl-1,3-thiazole sont préparés selon la description de M.C. Wilkes, P.B. Lavrik, J. Greenplate, J. Agric. Food Chem., 39, 1652 (1991). La source des autres produits chimiques est indiquée dans le texte.

Préparation 1 : acide (±)-5-[c-2-(3,4-dibromophényl)-r-1-fluorocyclopropyl]-penta-2,4-diénoïque

(i) On prépare le 3-(3,4-dibromophényl-2-fluoroprop-2-énoate de (Z)-éthyle d'une manière analogue à l'exemple 24 de EP-A1-0 369 762, à partir du 3,4-dibromobenzaldéhyde (exemple 14 de EP-A1-0 369 762). RMN de $^1H$ : 1,2 (3H, t), 4,3 (2H, q), 6,6 (1H, d), 7,4 (3H, m).

(ii) On prépare le (+)-5-[c-2-(3,4-dibromophényl)-r-1-fluorocyclopropyl]penta-2,4-diénoate d'éthyle à partir du composé ci-dessus par un procédé analogue à l'exemple 1 de EP-A-0369762 en utilisant du 4-phosphonocrotonate de triéthyle (de Lancaster). RMN de $^1H$ : 1,3 (3H, t), 1,6 (1H, m), 2,3 (1H, m), 4,2 (2H, q), 5,8 (1H, d), 5,9 (1H, dd), 6,4 (1H, dd), 7,0 (1H, m), 7,3 (1H, dd), 7,5 (2H, m).

(iii) On agite et on chauffe à 50°C le (±)-5-[c-2-(3,4-dibromophényl)-r-1-fluorocyclopropyl]-penta-2,4-diénoate d'éthyle (3,5 g) dans de l'éthanol (50 ml). On ajoute une solution d'hydroxyde de sodium (0,8 g) dans de l'eau (5 ml) et on poursuit le chauffage pendant encore trois heures. On élimine le solvant

sous vide et on ajoute de l'eau et de l'éther diéthylique. La solution aqueuse est séparée et acidifiée avec de l'acide chlorhydrique dilué. Le précipité est extrait avec de l'éther diéthyligue, lavé avec de la saumure et séché sur du sulfate de magnésium. L'élimination du solvant sous vide donne l'acide (±)-5-[c-2-(3,4-dibromophényl)-r-1-fluorocyclopropyl]-penta-2,4-diénoïque (2,5 g). RMN de $^1H$ : 1,5 (1H, m), 1,8 (1H, m), 2,3 (1H, m), 5,9 (2H, m), 6,4 (1H, dd), 7,0 (1H, m), 7,3 (1H, dd), 7,5 (2H, m).

Exemple 1

(+)-(2E,4E)-N-(2-Thiazolyl)-5-[c-2-(3,4-dibromophényl)-r-1-fluorocyclopropyl]penta-2,4-diénamide

(iv) On met en suspension l'acide ci-dessus (350 mg) dans le dichlorométhane (15 ml) et on agite sous azote à la température ambiante tout en ajoutant du chlorure d'oxalyle (de Aldrich) (156 mg/107 µl) et du diméthylformamide (1 goutte). On poursuit l'agitation pendant deux heures et on élimine le solvant sous vide. On dissout le solide restant dans le dichloro-méthane (20 ml) et on ajoute de la pyridine (100 µl) et de la 2-amino-thiazole (de Aldrich). Après avoir agité pendant une nuit, on lave successivement la solution organique avec de l'acide chlorhydrique 2N, une solution saturée d'hydrogénocarbonate de sodium, de la saumure, et on la sèche sur du sulfate de magnésium. L'élimination du solvant sous vide donne un solide. La purification par chromatographie (silice, acétate d'éthyle) donne le composé du titre (160 mg). Ccm (silice, acétate d'éthyle) Rf 0,7,
F 181 °C. RMN (DMSO) de $^1H$ : 1,8 (1H, m), 2,1 (1H, m), 2,7 (1H, m), 6,5 (3H, m), 7,3 (2H, m), 7,5 (1H, m), 7,8 (2H, m), 8,4 (2H, m) , 9,9 (1H, s).

Exemple 2

(+)-(2E/Z,4E)-N-Pipéridino-5-[c-2-(3,4-dibromophényl)-r-1-fluorocyclopropyl]-3-méthylpenta-2,4-diénamide (2E:2Z = 9:1)

On prépare le composé du titre à partir de 1-aminopipéridine d'une façon analogue à l'exemple 1, mais en utilisant comme base de la triéthylamine à la place de la pyridine.

| Composé n° | Nom du composé |
|---|---|
| 3 | (±)-(2E,4E)-N-(2-pyridyl)-5-[c-2-(3,4-dibromophényl)-r-1-fluorocyclopropyl]-3-méthylpenta-2,4-diénamide |
| 6 | (±)-(2E,4E)-N-(3-méthyl-2-pyridyl)-5-[c-2-(3,4-dibromophényl)-r-1-fluorocyclopropyl]penta-2,4-diénamide |
| 7 | (±)-(2E,4E)-N-(2-thiazolyl)-5-[c-2-(3,4-dibromophényl)-r-1-fluorocyclopropyl]penta-2,4-diénamide |
| 8 | (±)-(2E,4E)-N-(5-méthylisoxazole-3-yl)-5-[c-2-(3,4-dibromophényl)-r-1-fluorocyclopropyl]penta-2,4-diénamide |
| 9 | (±)-(2E,4E)-N-(1,3,4-thiadiazole-2-yl)-5-[c-2-(3,4-dibromophényl)-r-1-fluorocyclopropyl]-penta-2,4-diénamide |
| 10 | (±)-(2E,4E)-N-(4-pyrimidyl)-5-[c-2-(3,4-dibromophényl)-r-1-fluorocyclopropyl]penta-2,4-diénamide |
| 11 | (±)-(2E,4E)-N-(3-pyrazolyl)-5-[c-2-(3,4-dibromophényl)-r-1-fluorocyclopropyl]penta-2,4-diénamide |
| 12 | (±)-(2E,4E)-N-(4-trifluorométhyl-1,3-thiazolyl)-5-[c-2-(3,4-dibromophényl)-r-1-fluorocyclopropyl]penta-2,4-diènamide |

13 (±)-(2E,4E)-N-2-(4-trifluorométhyl-5-chloro-1,3-thiazolyl)-5-[c-2-(3,4-dibromophényl)-r-1-fluorocyclopropyl]penta-2,4-diénamide

14 (±)-(2E/Z,4E)-N-pipéridino-5-[c-2-(3,4-dibromophényl)-r-1-fluorocyclopropyl]-3-méthylpenta-2,4-diénamide (2E/2Z = 9/1)

15 (±)-(2E,4E)-N-pipéridino-5-[c-2-(3,4-dibromophényl)-r-1-fluorocyclopropyl]penta-2,4-diénamide

16 (±)-(2E,4E)-N-(2,6-diméthylpipéridino)-5-[c-2-(3,4-dibromophényl)-r-1-fluorocyclopropyl]penta-2,4-diénamide

17 (±)-(2E,4E)-N-morpholino-5-[c-2-(3,4-dibromophényl)-r-1-fluorocyclopropyl]penta-2,4-diénamide

18 (±)-(2E,4E)-N-(perhydroazépine-1-yl)-5-[c-2-(3,4-dibromophényl)-r-1-fluorocyclopropyl]penta-2,4-diénamide

| Composé n° | Réactif de Wittig | Amine | EE:EZ a : b | Préparé selon l 'exemple No. |
|---|---|---|---|---|
| 3 | 2 | 2-Aminopyridine | a | 1 |
| 6 | 1 | 2-Amino-3-methylpyridine | a | 1 |
| 7 | 1 | 2-Aminothiazole | a | 1 |
| 8 | 1 | 3-Amino-5-methylisoxazole | a | 1 |
| 9 | 1 | 2-Amino-1,3,4-thiadiazole | a | 1 |
| 10 | 1 | 4-Aminopyrimidine | a | 1 |
| 11 | 1 | 3-Aminopyrazole | a | 1 |
| 12 | 1 | 2-Amino-4-trifluoromethyl-1,3-thiazole | a | 1 |
| 13 | 1 | 2-Amino-5-chloro-4-trifluoromethyl-1,3-thiazole | a | 1 |
| 14 | 2 | 1-Aminopiperidine | 9:1 | 2 |
| 15 | 1 | 1-Aminopiperidine | a | 2 |
| 16 | 1 | 1-Amino-2,6-dimethylpiperidine | a | 2 |
| 17 | 1 | 4-Aminomorpholine | a | 2 |
| 18 | 1 | N-aminohexamethyleneimine | a | 2 |
| Réactif de Wittig<br>1 = 4-phosphonocrotonate de triéthyle<br>2 = 3-méthyl-4-phosphonocrotonate de triéthyle | | | | |

| Composé n° | Pt de F °C | spectre de masse M + 1 |
|---|---|---|
| 3 | 155-6 | 479 |
| 6 | 96-9o | 478 |
| 7 | 201.5 | 471 |
| 8 | 190-2 | 469 |
| 9 | 205.3. | 472 |
| 10 | 154.1 | 466 |
| 11 | 52.7 | 454 |
| 12 | 178-80 | 539 |
| 13 | 150.5 | 573 |
| 14 | 60.5 | 487 |
| 15 | 175-6 | 473 |
| 16 | 178.9 | 499 |
| 17 | 179.5 | 475 |
| 18 | 165.2 | 485 |

RESULTATS BIOLOGIQUES

Les exemples suivants illustrent, sans la limiter, l'activité pesticide des composés de formule (I)

EXEMPLE A - ESSAIS DE VAPORISATION

On étudie l'activité de composés de l'invention en dissolvant les composés dans de l'acétone (5%) et ensuite en diluant dans un système eau "Synperonic" (94,5%:0,5%) pour obtenir une émulsion aqueuse. On utilise ensuite la solution pour traiter les insectes suivants, pour lesquels on observe l'activité aux taux de vaporisation suivants:

Plutella xylostella

Des disques foliaires de choux de Chine, infestés de 8 larves de Plutella au second stade larvaire, sont vaporisés avec la solution contenant le composé. La mortalité est évaluée au bout de 2 jours à 25°C.

Les composés suivants sont actifs à 1000 ppm ou moins : 3, 6, 8.

Les composés suivants sont actifs à 200 ppm ou moins : 15, 16, 17, 18.

Spodoptera littoralis

On vaporise sur des feuilles non infestées la solution étudiée contenant le composé et on les laisse sécher. On les infeste ensuite avec 10 larves venant d'éclore. La mortalité est évaluée au bout de 3 jours.

Les composés suivants sont actifs à 1000 ppm ou moins : 3, 9.

Les composés suivants sont actifs à 200 ppm ou moins : 14, 15, 16, 17, 18.

Diabrotica undecimpunctata

Sur du papier filtre et de la nourriture, on vaporise la solution étudiée, puis on les infeste avec 8 larves au second stade larvaire. On évalue l'activité au bout de 2 jours.

Les composés suivants sont actifs à 1000 ppm ou moins : 3, 14, 15, 16, 17, 18.

Tetranychus urticae

Des disques foliaires de haricots verts infestés de stades mélangés d'acariens sont aspergées de la solution contenant le composé. La mortalité est évaluée au bout de 2 jours.

Les composés suivants sont actifs à 1000 ppm ou moins : 14, 18.

Myzus perticae

Des disques foliaires de choux de Chine infestés avec 10 adultes Myzus sont vaporisés avec la solution contenant le composé. La mortalité est évaluée au bout de 2 jours.

Les composés suivants sont actifs à 1000 ppm ou moins : 14, 16.

EXEMPLE B - ESSAIS D'APPLICATION EN TOPIOUE

Blatella germanica

On applique en topique 0,5 µl d'une solution du composé dans la butanone (avec ou sans butylate de pipéronyle) sur des mâles B. germanica. La mortalité est évaluée au bout de 6 jours.

Les composés suivants sont actifs à raison de 10 µg ou moins (+ butoxyde de pipéronyle) : 14, 15, 17.

Musca domestica

On applique en topique 0,3 µl d'une solution du composé dans la butanone (avec butylate de pipéronyle) sur des femelles M. domestica. La mortalité est évaluée au bout de 2 jours.

Les composés suivants sont actifs à raison de 1,5 µg ou moins : 3, 10, 14, 15, 16, 17, 18.

| Composé n° | Résultats de RMN de $^1H$ |
|---|---|
| 3 | 1.5 (1H,m), 1.8 (1H,m), 2.3 (1H,m), 2.4 (3H,s), 5.8 (2H,m), 6.4 (1H,d), 7.0 (2H,m), 7.5 (2H,m), 7.7 (1H,m), 8.3 (2H,m), 9.6 (1H,s). |
| 6 | 1.5 (1H,m), 1.8 (1H,m), 2.2 (3H,s), 2.3 (1H,m), 5.9 (1H,dd), 6.2 (1H,d), 6.4 (1H,dd), 7.0 (1H,dd), 7.5 (6H,m), 8.4 (1H,m). |
| 7* | 1.8 (1H,m), 2.2 (1H,m), 2.7 (1H,m), 6.5 (3H,m), 7.3 (2H,m), 7.5 (1H,dd), 7.6 (1H,d), 7.8 (2H,m), 13.0 (1H,s). |
| 8 | 1.6 (1H,m), 1.8 (1H,m), 2.3 (1H,m), 2.4 (3H,s), 5.9 (1H,dd), 6.1 (1H,d), 6.5 (1H,dd), 6.8 (1H,s), 7.0 (1H,dd), 7.4 (1H,dd), 7.5 (2H,m), 8.9 (1H,s). |

| | |
|---|---|
| 9* | 1.5 (1H,m), 1.9 (1H,m), 2.4 (1H,m), 6.2 (4H,m), 7.0 (1H,dd), 7.2 (1H,dd), 7.5 (2H,m), 9.0 (1H,s). |
| 10 | 1.6 (1H,m), 1.8 (1H,m), 2.3 (1H,m), 6.0 (2H,m), 6.5 (1H,dd), 7.0 (1H,dd), 7.5 (3H,m), 8.2 (2H,m), 8.6 (1H,m), 8.9 (1H,s). |
| 11 | 1.5 (1H,m), 1.8 (1H,m), 2.3 (1H,m), 5.6 (1H,b), 6.0 (2H,m), 6.6 (1H,dd), 7.0 (1H,dd), 7.3 (5H,m), 8.1 (1H,d). |
| 12* | 1.5 (1H,m), 1.8 (1H,m), 2.3 (1H,m), 5.8 (1H,dd), 6.2 (1H,d), 6.5 (1H,dd), 7.0 (1H,dd), 7.3 (1H,s), 7.4 (1H,dd), 7.5 (3H,m). |
| 13 | 1.6 (1H,m), 1.8 (1H,m), 2.4 (1H,m), 6.0 (2H,m), 6.5 (1H,dd), 7.0 (1H,dd), 7.3 (1H,m), 7.5 (2H,m), 9.0 (1H,s). |
| 14 | 1.5 (3H,m), 2.8 (7H,m), 2.0 and 2.3 (3H,s), 2.5 (3H,m), 5.6-6.5 (3H,m), 7.0 (1H,m), 7.5 (2H,m). |
| 15 | 1.5 (1H,m), 2.8 (10H,m), 2.3 (3H,m), 5.9 (1H,dd), 6.3 (1H,s), 6.5 (1H,dd), 6.8 (1H,d), 7.0 (1H,m), 7.3 (1H,dd), 7.5 (2H,m). |
| 16 | 1.0 (6H,m), 2.7 (8H,m), 2.3 (3H,m), 5.8 (1H,m), 6.6 (2H,m), 7.0 (1H,m), 7.3 (1H,m), 7.5 (2H,m). |
| 17* | 1.6 (1H,m), 1.9 (1H,m), 2.4 (1H,m), 2.8 (4H,m), 3.6 (4H,m), 5.8 (1H,dd), 6.2 (1H,m), 6.4 (1H,m), 7.0 (2H,m), 7.5 (2H,m), 8.5 (1H,b). |
| 18 | 1.7 (10H,m), 2.3 (1H,m), 3.0 (4H,m), 5.8 |

(1H,m), 6.4 (1H,m), 6.8 (1H,d), 7.0 (1H,dd), 7.3 (1H,dd), 7.5 (2H,m).

* désigne des spectres de RMN de $^{1}H$ obtenus en solution dans le $d_6$-diméthylsulfoxyde.

## Revendications

1. Un composé de formule (I) :

$$Q(CH_2)_a(O)_bQ_1CR_2=CR_3CR_4=CR_5CX_1NHR_1$$

ou l'un de ses sels, dans lequel Q est un système à noyau monocyclique ou un système à noyau bicyclique condensé, éventuellement substitués, dans lequel au moins un noyau est aromatique ou Q est un groupe dihalogénovinyle ou un groupe $R_6C{\equiv}C$-, dans lequel $R_6$ est $C_{1-4}$-alkyle, tri-$C_{1-4}$-alkylsilyle, halogène ou hydrogène;
$Q_1$ est un noyau 1,2-cyclopropyle, éventuellement substitué par un ou plusieurs groupe(s) choisi(s) parmi $C_{1-3}$-alkyle, halogène, $C_{1-3}$-halogénoalkyle, $C_{1-3}$-alkynyle, ou cyano; ou $Q^1$ est $(CH_2)_m$, où m = 1 à 7; a = 0 ou 1; b = 0 ou 1;
$R_2$, $R_3$, $R_4$ et $R_5$ sont identiques ou différents, l'un d'eux au moins étant hydrogène et les autres étant choisis indépendamment parmi hydrogène, halogène, $C_{1-4}$-alkyle ou $C_{1-4}$-halogénoalkyle;
$X_1$ est oxygène ou soufre;
$R_1$ est un noyau hétérocyclique éventuellement substitué aromatique, partiellement saturé ou saturé, a cinq ou six chainons, contenant au moins un atome d'azote et éventuellement jusqu'à trois autres hétéroatomes choisis parmi, l'azote, l'oxygène ou le soufre, et qui se rattache à l'azote de la fonction amide par un atome d'azote ou par un carbone adjacent à un atome d'azote, ou $R_1$ est le radical 1-azépinyle.

2. Un composé de formule (I) ou l'un de ses sels selon la revendication 1, dans lequel Q est un groupe phényle, pyridyle, thiényle, naphtyle, quinoléinyle, tétrahydronaphtyle ou indanyle, chacun éventuellement substitué par
   a) un radical $C_{1-6}$-hydrocarboné, $C_{1-6}$-alkoxy ou méthylènedioxy, chacun étant éventuellement substitué par un à cinq atome(s) d'halogène; ou
   b) un atome d'halogène, un radical cyano, nitro; ou
   c) un groupe $S(O)_nR_7$ dans lequel n = 0, 1 ou 2 et $R_7$ est $C_{1-6}$-alkyle, éventuellement substitué par un ou plusieurs atomes d'halogène ou $R_7$ est amino, éventuellement substitué par un ou deux groupe(s) $C_{1-6}$-alkyle; ou
   d) un groupe $NR_8R_9$, dans lequel $R_8$ et $R_9$ sont indépendamment choisis parmi hydrogène, $C_{1-6}$-alkyle ou un groupe $COR_{12}$, dans lequel $R_{12}$ est $C_{1-6}$-alkyle ou $C_{1-6}$-alkoxy.

3. Un composé de formule (I) ou l'un de ses sels selon la revendication 1 ou 2, dans lequel $R_1$ est un groupe pyrrole, pyrazole, imidazole, thiazole, oxazole, isoxazole, isothiazole, triazole, pipéridine, morpholine ou chacun éventuellement substitué par 1 à 5 substituant(s) choisi(s) parmi: $C_{1-4}$-alkyle et $C_{1-4}$-alkoxy, chacun éventuellement substitué par 1 à 5 atome(s) d'halogène; halogène, cyano, $C_{1-3}$-alkynyle, $C_{1-3}$-alkényle, nitro, un groupe $S(O)_nR_7$, dans lequel n = 0, 1 ou 2 et $R_7$ est $C_{1-4}$-alkyle, éventuellement substitué par 1 à 5 halogène(s), un groupe $NR_8R_9$, dans lequel $R_8$ et $R_9$ sont individuellemment choisis parmi hydrogène ou $C_{1-4}$-alkyle, un groupe $=X_2$ dans lequel $X_2$ représente O, S ou $NR_{10}$ (dans lequel $R_{10}$ est choisi parmi hydrogène, $C_{1-4}$-alkyle, $C_{1-4}$-alkoxy et $COR_{11}$ dans lequel $R_{11}$ est $C_{1-4}$-alkyle).

4. Un composé de formule (I) ou l'un de ses sels selon l'une quelconque des revendications 1 à 3, dans lequel, indépendamment l'un de l'autre, $R_2$ représente l'hydrogène; $R_3$ représente hydrogène ou fluoro; $R_5$ représente hydrogène ou fluoro; et $R_4$ représente hydrogène ou $C_{1-4}$-alkyle.

**5.** Un composé de formule (I) ou l'un de ses sels selon l'une quelconque des revendications 1 à 4, dans lequel $Q_1$ est un groupe 1,2-cyclopropyle, non substitué aux positions 2- et 3- et substitué ou non substitué par fluoro ou chloro à la position 1-; b = 0; et a = 0.

**6.** Un composé de formule (I) ou l'un de ses sels selon l'une quelconque des revendications 1 à 4, dans lequel $Q_1$ est un groupe $(CH_2)m$; b = 1; m = 6 quand a = 1 ou m = 7 quand a = 0.

**7.** Composés de formule (I) ou l'un de leurs sels selon la revendication 1 correspondant à un composé de formule (II):

$$Q_aQ_{1a}CR_{2a} = CR_{3a}CR_{4a} = CR_{5a}C(=X_{1a})\ NHR_{1a} \qquad (II)$$

dans laquelle $Q_a$ est un groupe phényle ou pyridyle éventuellement substitué ou un système à noyau condensé bicyclique, éventuellement substitué, dont au moins un noyau est aromatique et contenant 0 ou 1 atome d'azote ou 0 ou 1 atome de soufre;
$Q_{1a}$ est un noyau 1,2-cyclopropyle, éventuellement substitué par un ou plusieurs groupes choisis parmi $C_{1-3}$-alkyle, halogène, ou $C_{1-3}$-halogènoalkyle;
$R_{2a}$, $R_{3a}$, $R_{4a}$ et $R_{5a}$ sont identiques ou différents, l'un d'eux au moins étant l'hydrogène et les autres étant choisis individuellemment parmi hydrogène, halogène, $C_{1-4}$-alkyle et $C_{1-4}$-halogènoalkyle;
$X_{1a}$ est l'oxygène ou le soufre;
et $R_{1a}$ est tel que défini dans la revendication 1 pour $R_1$.

**8.** Composés de formule (II) selon la revendication 7, dans lesquels $Q_a$ est un groupe phényle, pyridyle ou naphtyle, chacun éventuellement substitué par
a) $C_{1-6}$-alkyle, $C_{1-6}$-alkoxy ou méthylènedioxy, chacun éventuellement substitué par un ou plusieurs atome(s) d'halogène; ou
b) halogène, cyano, nitro; ou
c) un groupe $S(O)_nR_{7a}$, dans lequel n = 0, 1 ou 2 et $R_{7a}$ est $C_{1-6}$-alkyle, éventuellement substitué par un atome d'halogène.

**9.** Composés de formule (II) ou l'un de leurs sels selon la revendication 7 ou la revendication 8 correspondant à un composé de formule (III)

$$Q_aQ_{1a}CH=CHCR_{4a}=CHCONHR_{1a} \qquad (III)$$

dans laquelle $Q_a$ est défini comme dans la revendication 7 ou la revendication 8 et $Q_{1a}$, $R_{4a}$ et $R_{1a}$ sont définis comme dans la revendication 7.

**10.** Composés de formule (I) ou l'un de leurs sels, selon la revendication 1 correspondant à un composé de formule (IV)

$$Q\ Q_1CH=CR_3CR_4=CR_5CONHR_1 \qquad (IV)$$

dans laquelle Q, $Q_1$, $R_4$ et $R_1$ sont définis comme dans la revendication 1.

**11.** Composés de formule (IV) selon la revendication 10, dans lesquels Q est phényle substitué; $Q_1$ est un noyau <u>trans</u> 1,2-cyclopropyle, où la position 2- du noyau cyclopropyle est non substituée ou substituée par fluoro ou chloro; $R_4$ est méthyle ou hydrogène; $R_3$ et $R_5$ sont individuellement hydrogène ou fluoro; et $R_1$ est défini comme dans la revendication 1.

**12.** Composés de formule (I) selon la revendication 1 choisis parmi:
(±)-(2E,4E)-N-(2-pyridyl)-5-[c-2-(3,4-dibromophényl)-r-l-fluorocyclopropyl]-3-méthylpenta-2,4-diènamide;
(±)-(2E,4E)-N-(3-méthyl-2-pyridyl)-5-[c-2-(3,4-dibromophényl)-r-1-fluorocyclopropyl]penta-2,4-diènamide;
(±)-(2E,4E)-N-(2-thiazolyl)-5-[c-2-(3,4-dibromophényl)-r-1-fluorocyclopropyl]penta-2,4-diènamide;
(±)-(2E,4E)-N-(5-méthylisoxazole-3-yl)-5-[c-2-(3,4-dibromophényl)-r-1-fluorocyclopropyl]penta-2,4-diènamide;
(±)-(2E,4E)-N-(1,3,4-thiadiazol-2-yl)-5-[c-2-(3,4-dibromophényl)-r-1-fluorocyclopropyl]penta-2,4-diènamide;

(±)-(2E,4E)-N-(4-pyrimidyl)-5-[c-2-(3,4-dibromophényl)-r-1-fluorocyclopropyl]penta-2,4-diènamide;
(±)-(2E,4E)-N-(3-pyrazolyl)-5-[c-2-(3,4-dibromophényl)-r-1-fluorocyclopropyl]penta-2,4-diènamide;
(±)-(2E,4E)-N-(4-trifluorométhyl-1,3-thiazolyl)-5-[c-2-(3,4-dibromophényl)-r-1-fluorocyclopropyl]penta-2,4-diènamide;
(±)-(2E,4E)-N-2-(4-trifluorométhyl-5-chloro-1,3-thiazolyl)-5-[c-2-(3,4-dibromophényl)-r-1-fluorocyclopropyl]penta-2,4-diènamide;
(±)-(2E/Z,4E)-N-pipéridino-5-[c-2-(3,4-dibromophényl)-r-1-fluorocyclopropyl]penta-2,4-diènamide;
(±)-(2E,4E)-N-pipéridino-5-[c-2-(3,4-dibromophényl)-r-1-fluorocyclopropyl]penta-2,4-diènamide;
(±)-(2E,4E)-N-(2,6-diméthylpipéridino)-5-[c-2-(3,4-dibromophényl)-r-1-fluorocyclopropyl]penta-2,4-diènamide;
(±)-(2E,4E)-N-morpholino-5-[c-2-(3,4-dibromophényl)-r-1-fluorocyclopropyl]penta-2,4-diènamide;
(±)-(2E,4E)-N-(perhydroazépin-1-yl)-5-[c-2-(3,4-dibromophényl)-r-1-fluorocyclopropyl]penta-2,4-diènamide;
et leurs sels.

**13.** Un procédé pour la préparation d'un composé selon l'une quelconque des revendications 1 à 12 qui comprend: soit

a) quand $X_1$ est oxygène, la réaction de l'acide ou du dérivé d'acide correspondant de formule (V)

$$Q(CH_2)_a(O)_bQ_1CR_2=CR_3CR_4=CR_5C(=X)Z_1 \qquad (V)$$

avec une amine $H_2NR_1$, dans laquelle Q, a, b, $Q_1$, $R_2$, $R_3$, $R_4$, $R_5$ et $R_1$ sont définis comme dans la revendication 1 et X est oxygène et $Z^1$ est hydroxy, $C_{1-6}$-alkoxy, halogène ou ester phosphoroimidate (-P(O)(O-aryl)NH-aryl où aryle est $C_{1-6}$-aryle); soit

b) la formation de la partie $-CR_2=CR_3CR_4=CR_5C(=X_1)NHR_1$ du composé de formule (I) par l'intermédiaire d'une réaction de type Wittig;

et éventuellement la transformation ultérieure de l'un des composés de formule (I) en un autre composé de formule (I) par des méthodes bien connues des hommes de métier.

**14.** Un procédé selon la revendication 13, dans lequel en a) le composé de formule (V), dans laquelle $Z_1$ est hydroxy et Q, $Q_1$, $R_2$, $R_3$, $R_4$, $R_5$, a et b sont comme définis dans la revendication 1, est préparé par hydrolyse de l'ester correspondant, dans lequel $Z_1$ est $C_{1-6}$-alkoxy et Q, $Q_1$, $R_2$, $R_3$, $R_4$, et $R_5$, a et b sont comme définis dans la revendication 1.

**15.** Un procédé selon la revendication 14, dans lequel le composé ester de formule (V), dans lequel $Z_1$ est $C_{1-6}$-alkoxy et Q, $Q_1$, $R_2$, $R_3$, $R_4$, $R_5$, a et b sont comme définis dans la revendication 1, est préparé par un procédé qui comprend soit

i) la réaction d'un composé de formule (VI)

$$Z_2CHR_3CR_4=CR_5COZ_1 \qquad (VI)$$

dans laquelle $R_3$, $R_4$, $R_5$ et $Z_1$ sont tels que définis précédemment et $Z_2$ représente un groupe $(aryl)_3P$, $(aryl)_2P(O)$ ou $(C_{1-4}\text{-alkoxy})_2P(O)$, avec un composé de formule (VII)

$$Q(CH_2)_a(O)_bQ_1CR_2=O \qquad (VII)$$

dans laquelle Q, $Q_1$, $R_2$, a et b sont tels que définis précédemment; soit

ii) le réarrangement et l'élimination de $HS(O)Z_3$ d'un composé de formule (VIII)

$$Q(CH_2)_a(O)_bQ_1CHR_2CHR_3CR_4=C\begin{matrix} / \; S(O)Z_3 \\ \backslash \; CO_2Z_4 \end{matrix} \qquad (VIII)$$

dans laquelle Q, $Q_1$, $R_2$, $R_3$, $R_4$, $Z_1$, a et b sont tels que définis précédemment et $Z_3$ est un groupe approprié quelconque, de préférence phényle, phényle substitué ou $C_{1-4}$-alkyle; soit
iii) l'élimination de $HOZ_5$ d'un composé de formule (IX)

$Q(CH_2)_a(O)_bQ_1CHR_2CR_3(OZ_5)CR_4=CR_5\text{-}COZ_1$ (IX)

dans laquelle Q, $Q_1$, $R_2$, $R_3$, $R_4$, $R_5$, $Z_1$, a et b sont tels que définis précédemment et $Z_5$ et hydrogène ou $C_{1-4}$-acyle; soit
(iv) la réaction d'un composé de formule (X)

$Q(CH_2)_a(O)_bQ_1CR_2=CR_3C(=O)R_4$ (X)

dans laquelle Q, $Q_1$, $R_2$, $R_3$, $R_4$, a et b sont tels définis précédemment, avec un composé de formule (XI)

$Me_3SiCHR_5COZ_1$ (XI)

dans laquelle $R_5$ et $Z_1$ sont comme définis précédemment; soit
v) la réaction d'un composé de formule (XII)

$Q(CH_2)_a(O)_bQ_1CR_2=CR_3C(OZ_6)=CR_5COZ_1$ (XII)

dans laquelle Q, $Q_1$, $R_2$, $R_3$, $R_5$, $Z_1$, a et b sont tels que définis précédemment et $Z_6$ est un groupe quelconque approprié, préférablement un groupe dialkylphosphate ou trifluorométhanesulfonate, avec un composé de formule (XIII)

$R^4M^1$ (XIII)

dans laquelle $R_4$ est défini comme précédemment, et M est un métal, préférablement le cuivre (I) ou le cuivre (I) associé avec du lithium ou du magnésium; soit
vi) la réaction d'un composé de formule (XIV)

$Q(CH_2)_a(O)_bQ_1CR_2=CR_3M_2$ (XIV)

dans laquelle Q, $Q_1$, $R_2$, $R_3$, a et b sont définis tels que précédemment et $M_2$ est un groupe contenant silyl-, préférablement triméthylsilyl-, ou un groupe contenant un métal, préférablement un groupe contenant du zirconium, de l'étain, de l'aluminium ou du zinc, plus préférablement un groupe chlorure de bis(cyclopentadiènyl)zirconium, avec un composé de formule (XV)

$YCR_4=CR_5COZ_1$ (XV)

dans laquelle $R_4$, $R_5$ et $Z_1$ sont définis comme précédemment et Y est halogène ou étain; soit
vii) l'élimination de $Z_3S(O)H$ d'un composé de formule (XVI)

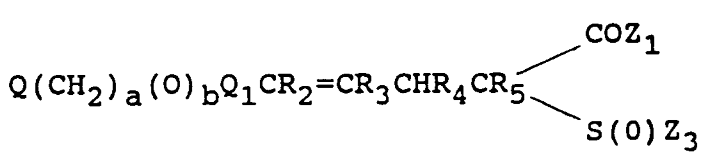

(XVI)

dans laquelle Q, $Q_1$, $R_2$, $R_3$, $R_4$, $R_5$, $Z_1$, a, b et $Z_3$ sont définis comme précédemment.

**16.** Un procédé selon la revendication 13, dans lequel en b) le composé de formule (I), dans laquelle Q, $Q_1$, $R_2$, $R_3$, $R_4$, $R_5$, a, b, $R_1$ et X sont définis comme dans la revendication 1, est préparé par un procédé qui comprend soit
i) la réaction d'un composé de formule (XVII)

$Q(CH_2)_a(O)_bQ_1CR_2=CR_3CR_4=O$ (XVII)

dans laquelle Q, $Q_1$, $R_2$, $R_3$, $R_4$, a et b sont définis comme précédemment, avec un composé de formule (XVIII)

$Z_2CHR_5C(=X)NHR_1$ (XVIII)

dans laquelle $R_1$, X et $R_5$ sont définis comme précédemment et $Z_2$ représente un groupe $(aryl)_3P$, $(aryl)_2P(O)$ ou $(C_{1-4}\text{-alkoxy})_2P(O)$; soit
ii) la réaction d'un composé de formule (XIX)

$Q(CH_2)_a(O)_bQ_1COR_2$ (XIX)

dans laquelle Q, $Q_1$, $R_2$, a et b sont définis comme précédemment, avec un composé de formule (XX)

$Z_2CHR_3CR_4=CR_5C(=X)NHR_1$ (XX)

dans laquelle $Z_1$, $R_3$, $R_4$, $R_5$, X et $R_1$ sont définis comme précédemment; soit
iii) la réaction d'un composé de formule (XXI)

$Q(CH_2)_a(O)Q_{1b}CR_2=CR_3CHR_5Z_2$ (XXI)

dans laquelle Q, $Q_1$, $R_2$, $R_3$, $R_5$ , $Z_2$, a et b sont comme définis précédemment, avec un composé de formule (XXII)

$R_5COC(=X)NHR_1$ (XXII)

dans laquelle $R_5$, X et $R_1$ sont définis comme précédemment.

**17.** Une composition pesticide comprenant un composé de formule (I) comme définie dans l'une quelconque des revendications 1 à 12, en mélange avec un ou plusieurs supports, diluants ou excipients.

**18.** Une composition pesticide à effet synergique comprenant un composé de formule (I) comme définie dans l'une quelconque des revendications 1 à 12, un synergiste pour le composé de formule (I) et un ou plusieurs supports, diluants et excipients.

**19.** Un mélange de l'un ou de plusieurs composés de formule (I), comme défini(s) dans l'une quelconque des revendications 1 à 12, et d'un autre composé pesticide.

**20.** Une méthode pour le contrôle des parasites, qui comprend l'application d'une quantité efficace d'un composé selon l'une quelconque des revendications 1 à 12 ou une composition ou mélange selon l'une quelconque des revendications 17 à 19, aux parasites ou à l'environnement susceptible d'infestation parasite.

**21.** Un composé de formule (I) selon l'une quelconque des revendications 1 à 12, ou une composition ou mélange selon l'une quelconque des revendications 17 à 19, pour l'utilisation en thérapie mise en oeuvre sur le corps humain ou animal.

## Claims

**1.** A compound of formula (I):

$Q(CH_2)_a(O)_bQ_1CR_2=CR_3CR_4=CR_5CX_1NHR_1$

or one of its salts, in which Q is a system with a monocyclic nucleus or a system with a condensed bicyclic nucleus, optionally substituted, in which at least one nucleus is aromatic or Q is a dihalogenovinyl group or an $R_6C{\equiv}C$- group, in which $R_6$ is $C_{1-4}$-alkyl, tri-$C_{1-4}$-alkylsilyl, halogen or

hydrogen;
$Q_1$ is a 1,2-cyclopropyl nucleus, optionally substituted by one or more groups chosen from $C_{1-3}$-alkyl, halogen, $C_{1-3}$-halogenoalkyl, $C_{1-3}$-alkynyl, or cyano; or $Q_1$ is $(CH_2)_m$, where m = 1 to 7; a = 0 or 1; b = 0 or 1;
$R_2$, $R_3$, $R_4$ and $R_5$ are identical or different, at least one of them being hydrogen and the others being chosen independently from hydrogen, halogen, $C_{1-4}$-alkyl or $C_{1-4}$-halogenoalkyl;
$X_1$ is oxygen or sulphur;
$R_1$ is an optionally substituted, partially saturated or saturated, aromatic heterocyclic nucleus with five or six members, containing at least one nitrogen atom and optionally up to three other heteroatoms chosen from nitrogen, oxygen or sulphur, and which is attached to the nitrogen of the amide function by a nitrogen atom or by a carbon adjacent to a nitrogen atom, or $R_1$ is the 1-azepinyl radical.

**2.** A compound of formula (I) or one of its salts according to claim 1, in which Q is a phenyl, pyridyl, thienyl, naphthyl, quinolinyl, tetrahydronaphthyl or indanyl group, each optionally being substituted by
a) a $C_{1-6}$-hydrocarbon, $C_{1-6}$-alkoxy or methylenedioxy, each being optionally substituted by one to five halogen atoms; or
b) a halogen atom, a cyano, nitro radical; or
c) an $S(O)_nR_7$ group in which n = 0, 1 or 2 and $R_7$ is $C_{1-6}$-alkyl, optionally substituted by one or more halogen atoms, or $R_7$ is amino, optionally substituted by one or two $C_{1-6}$-alkyl groups; or
d) an $NR_8R_9$ group, in which $R_8$ and $R_9$ are chosen independently from hydrogen, $C_{1-6}$-alkyl, or a $COR_{12}$ group, in which $R_{12}$ is $C_{1-6}$-alkyl or $C_{1-6}$-alkoxy.

**3.** A compound of formula (I) or one of its salts according to claim 1 or 2, in which $R_1$ is a pyrrole, pyrazole, imidazole, thiazole, oxazole, isoxazole, isothiazole, triazole, piperidine or morpholine group, each optionally substituted by 1 to 5 substituents chosen from: $C_{1-4}$-alkyl and $C_{1-4}$-alkoxy, each optionally substituted by 1 to 5 halogen atoms, halogen, cyano, $C_{1-3}$-alkynyl, $C_{1-3}$-alkenyl, nitro, an $S(O)_nR_7$ group, in which n = 0, 1 or 2 and $R_7$ is $C_{1-4}$-alkyl, optionally substituted by 1 to 5 halogens, an $NR_8R_9$ group, in which $R_8$ and $R_9$ are individually chosen from hydrogen or $C_{1-4}$-alkyl, an $=X_2$ group in which $X_2$ represents O, S or $NR_{10}$ (in which $R_{10}$ is chosen from hydrogen, $C_{1-4}$-alkyl, $C_{1-4}$-alkoxy and $COR_{11}$ in which $R_{11}$ is $C_{1-4}$-alkyl).

**4.** A compound of formula (I) or one of its salts according to any one of claims 1 to 3, in which, independently of each other, $R_2$ represents hydrogen; $R_3$ represents hydrogen or fluoro; $R_5$ represents hydrogen or fluoro; and $R_4$ representshydrogen or $C_{1-4}$-alkyl.

**5.** A compound of formula (I) or one of its salts according to any one of claims 1 to 4, in which $Q_1$ is a 1,2-cyclopropyl group, not substituted in positions 2- and 3- and substituted or not substituted by fluoro or chloro in position 1-; b = 0; and a = 0.

**6.** A compound of formula (I) or one of its salts according to any one of claims 1 to 4, in which $Q_1$ is a $(CH_2)_m$ group; b = 1; m = 6 when a = 1 or m = 7 when a = 0.

**7.** Compounds of formula (I) or one of their salts according to claim 1 corresponding to a compound of formula (II):

$$Q_aQ_{1a}CR_{2a} = CR_{3a}CR_{4a} = CR_{5a}C(=X_{1a})\ NHR_{1a} \qquad (II)$$

in which $Q_a$ is an optionally substituted phenyl or pyridyl group or an optionally substituted bicyclic condensed nucleus system, of which at least one nucleus is aromatic and containing 0 or 1 nitrogen atom or 0 or 1 sulphur atom;
$Q_{1a}$ is a 1,2-cyclopropyl nucleus, optionally substituted by one or more groups chosen from $C_{1-3}$-alkyl, halogen, or $C_{1-3}$-halogenoalkyl;
$R_{2a}$, $R_{3a}$, $R_{4a}$ and $R_{5a}$ are identical or different, at least one of them being hydrogen and the others being chosen individually from hydrogen, halogen, $C_{1-4}$-alkyl and $C_{1-4}$-halogenoalkyl;
$X_{1a}$ is oxygen or sulphur;
and $R_{1a}$ is as defined in claim 1 for $R_1$.

**8.** Compounds of formula (II) according to claim 7, in which $Q_a$ is a phenyl, pyridyl or naphthyl group, each optionally substituted by
a) $C_{1-6}$-alkyl, $C_{1-6}$-alkoxy or methylenedioxy, each optionally substituted by one or more halogen atoms; or
b) halogen, cyano, nitro; or
c) an $S(O)_nR_{7a}$ group, in which n = 0, 1 or 2 and $R_{7a}$ is $C_{1-6}$-alkyl, optionally substituted by a halogen atom.

**9.** Compounds of formula (II) or one of their salts according to claim 7 or claim 8 corresponding to a compound of formula (III):

$Q_aQ_{1a}CH=CHCR_{4a}=CHCONHR_{1a}$ (III)

in which $Q_a$ is defined as in claim 7 or claim 8 and $Q_{1a}$, $R_{4a}$ and $R_{1a}$ are defined as in claim 7.

**10.** Compounds of formula (I) or one of their salts, according to claim 1, corresponding to a compound of formula (IV):

$Q\,Q_1CH=CR_3CR_4=CR_5CONHR_1$ (IV)

in which Q, $Q_1$, $R_4$ and $R_1$ are defined as in claim 1.

**11.** Compounds of formula (IV) according to claim 10, in which Q is substituted phenyl; $Q_1$ is a 1,2-cyclopropyl trans nucleus, where position 2- of the cyclopropyl nucleus is not substituted or substituted by fluoro or chloro; $R_4$ is methyl or hydrogen; $R_3$ and $R_5$ are individually hydrogen or fluoro; and $R_1$ is defined as in claim 1.

**12.** Compounds of formula (I) according to claim 1 chosen from:
(±)-(2E,4E)-N-(2-pyridyl)-5-[c-2-(3,4-dibromophenyl)-r-1-fluorocyclopropyl]-3-methylpenta-2,4-dienamide;
(±)-(2E,4E)-N-(3-methyl-2-pyridyl)-5-[c-2-(3,4-dibromophenyl)-r-1-fluorocyclopropyl]penta-2,4-dienamide;
(±)-(2E,4E)-N-(2-thiazolyl)-5-[c-2-(3,4-dibromophenyl)-r-1-fluorocyclopropyl]penta-2,4-dienamide;
(±)-(2E,4E)-N-(5-methylisoxazole-3-yl)-5-[c-2-(3,4-dibromophenyl)-r-1-fluorocyclopropyl]penta-2,4-dienamide;
(±)-(2E,4E)-N-(1,3,4-thiadiazol-2-yl)-5-[c-2-(3,4-dibromophenyl)-r-1-fluorocyclopropyl]penta-2,4-dienamide;
(±)-(2E,4E)-N-(4-pyrimidyl)-5-[c-2-(3,4-dibromophenyl)-r-1-fluorocyclopropyl]penta-2,4-dienamide;
(±)-(2E,4E)-N-(3-pyrazolyl)-5-[c-2-(3,4-dibromophenyl)-r-1-fluorocyclopropyl]penta-2,4-dienamide;
(±)-(2E,4E)-N-(4-trifluoromethyl-1,3-thiazolyl)-5-[c-2-(3,4-dibromophenyl)-r-1-fluorocyclopropyl]penta-2,4-dienamide;
(±)-(2E,4E)-N-2-(4-trifluoromethyl-5-chloro-1,3-thiazolyl)-5-[c-2-(3,4-dibromophenyl)-r-1-fluorocyclopropyl]penta-2,4-dienamide;
(±)-(2E/Z,4E)-N-piperidino-5-[c-2-(3,4-dibromophenyl)-r-1-fluorocyclopropyl]penta-2,4-dienamide;
(±)-(2E,4E)-N-piperidino-5-[c-2-(3,4-dibromophenyl)-r-1-fluorocyclopropyl]penta-2,4-dienamide;
(±)-(2E,4E)-N-(2,6-dimethylpiperidino)-5-[c-2-(3,4-dibromophenyl)-r-1-fluorocyclopropyl]penta-2,4-dienamide;
(±)-(2E,4E)-N-morpholino-5-[c-2-(3,4-dibromophenyl)-r-1-fluorocyclopropyl]penta-2,4-dienamide;
(±)-(2E,4E)-N-(perhydroazepin-1-yl)-5-[c-2-(3,4-dibromophenyl)-r-1-fluorocyclopropyl]penta-2,4-dienamide;
and their salts.

**13.** A process for the preparation of a compound according to any one of claims 1 to 12 which comprises: either
a) when $X_1$ is oxygen, the reaction of the acid or of the corresponding acid derivative of formula (V):

$Q(CH_2)_a(O)_bQ_1CR_2=CR_3CR_4=CR_5C(=X)Z_1$ (V)

with an amine $H_2NR_1$, in which Q, a, b, $Q_1$, $R_2$, $R_3$, $R_4$, $R_5$ and $R_1$ are defined as in claim 1 and X is oxygen and $Z_1$ is hydroxy, $C_{1-6}$-alkoxy, halogen or ester phosphoroimidate (-P(O)(O-aryl)NH-aryl where aryl is $C_{1-6}$-aryl); or

b) the formation of the $-CR_2=CR_3CR_4=CR_5C(=X_1)NHR_1$ part of the compound of formula (I) by means of a Wittig type reaction;

and optionally the subsequent conversion of one of the compounds of formula (I) into another compound of formula (I) by methods which are well-known to a man skilled in the art.

**14.** A process according to claim 13, in which in a) the compound of formula (V), in which $Z_1$ is hydroxy and Q, $Q_1$, $R_2$, $R_3$, $R_4$, $R_5$, a and b are as defined in claim 1, is prepared by hydrolysis of the corresponding ester, in which $Z_1$ is $C_{1-6}$-alkoxy and Q, $Q_1$, $R_2$, $R_3$, $R_4$ and $R_5$, a and b are as defined in claim 1.

**15.** A process according to claim 14, in which the ester compound of formula (V), in which $Z_1$ is $C_{1-6}$-alkoxy and Q, $Q_1$, $R_2$, $R_3$, $R_4$, $R_5$, a and b are as defined in claim 1, is prepared by a process which comprises either

i) the reaction of a compound of formula (VI):

$Z_2CHR_3CR_4=CR_5COZ_1$ (VI)

in which $R_3$, $R_4$, $R_5$ and $Z_1$ are as defined previously and $Z_2$ represents an $(aryl)_3P$, $(aryl)_2P(O)$ or $(C_{1-4}\text{-alkoxy})_2P(O)$ group, with a compound of formula (VII):

$Q(CH_2)_a(O)_bQ_1CR_2=O$ (VII)

in which Q, $Q_1$, $R_2$, a and b are as defined previously; or

ii) the rearrangement and elimination of $HS(O)Z_3$ from a compound of formula (VIII):

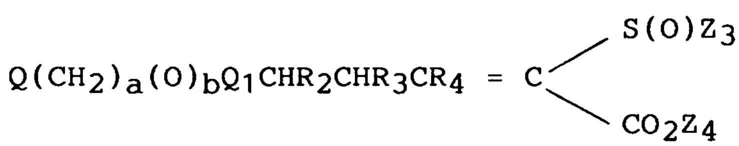

(VIII)

in which Q, $Q_1$, $R_2$, $R_3$, $R_4$, $Z_1$, a and b are as defined previously and $Z_3$ is any suitable group, preferably phenyl, substituted phenyl or $C_{1-4}$-alkyl; or

iii) the elimination of $HOZ_5$ from a compound of formula (IX):

$Q(CH_2)_a(O)_bQ_1CHR_2CR_3(OZ_5)CR_4=CR_5\text{-}COZ_1$ (IX)

in which Q, $Q_1$, $R_2$, $R_3$, $R_4$, $R_5$, $Z_1$, a and b are as defined previously and $Z_5$ is hydrogen or $C_{1-4}$-acyl; or

(iv) the reaction of a compound of formula (X):

$Q(CH_2)_a(O)_bQ_1CR_2=CR_3C(=O)R_4$ (X)

in which Q, $Q_1$, $R_2$, $R_3$, $R_4$, a and b are as defined previously, with a compound of formula (XI):

$Me_3SiCHR_5COZ_1$ (XI)

in which $R_5$ and $Z_1$ are as defined previously; or

(v) the reaction of a compound of formula (XII):

$Q(CH_2)_a(O)_bQ_1CR_2=CR_3C(OZ_6)=CR_5COZ_1$ (X)

in which Q, $Q_1$, $R_2$, $R_3$, $R_5$, $Z_1$, a and b are as defined previously and $Z_6$ is any suitable group, preferably a dialkylphosphate or trifluoromethanesulphonate group, with a compound of formula

(XIII):

$R_4M_1$ (XIII)

in which $R_4$ is defined as previously, and $M_1$ is a metal, preferably copper (I) or copper (I) combined with lithium or magnesium; or
(vi) the reaction of a compound of formula (XIV):

$Q(CH_2)_a(O)_bQ_1CR_2=CR_3M_2$ (XIV)

in which Q, $Q_1$, $R_2$, $R_3$, a and b are defined as previously and $M_2$ is a group containing silyl-, preferably trimethylsilyl-, or a group containing a metal, preferably a group containing zirconium, tin, aluminium or zinc, more preferably a bis(cyclopentadienyl)zirconium chloride group, with a compound of formula (XV):

$YCR_4=CR_5COZ_1$ (XV)

in which $R_4$, $R_5$ and $Z_1$ are defined as previously and Y is halogen or tin; or
(vii) the elimination of $Z_3S(O)H$ from a compound of formula (XVI):

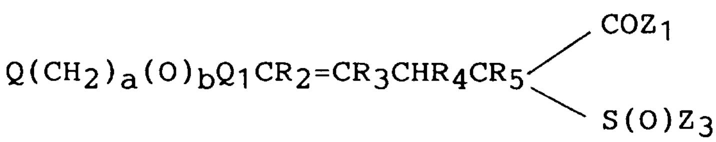

(XVI)

in which Q, $Q_1$, $R_2$, $R_3$, $R_4$, $R_5$, $Z_1$, a, b and $Z_3$ are defined as previously.

**16.** A process according to claim 13, in which in b) the compound of formula (I), in which Q, $Q_1$, $R_2$, $R_3$, $R_4$, $R_5$, a, b, $R_1$ and X are defined as in claim 1, is prepared by a process which comprises either
i) the reaction of a compound of formula (XVII):

$Q(CH_2)_a(O)_bQ_1CR_2=CR_3CR_4=O$ (XVII)

in which Q, $Q_1$, $R_2$, $R_3$, $R_4$, a and b are defined as previously, with a compound of formula (XVIII):

$Z_2CHR_5C(=X)NHR_1$ (XVIII)

in which $R_1$, X and $R_5$ are defined as previously and $Z_2$ represents an $(aryl)_3P$, $(aryl)_2P(O)$ or $(C_{1-4}\text{-alkoxy})_2P(O)$ group; or
ii) the reaction of a compound of formula (XIX):

$Q(CH_2)_a(O)_bQ_1COR_2$ (XIX)

in which Q, $Q_1$, $R_2$, a and b are defined as previously, with a compound of formula (XX):

$Z_2CHR_3CR_4=CR_5C(=X)NHR_1$ (XX)

in which $Z_1$, $R_3$, $R_4$, $R_5$, X and $R_1$ are defined as previously; or
iii) the reaction of a compound of formula (XXI):

$Q(CH_2)_a(O)Q_{1b}CR_2=CR_3CHR_5Z_2$ (XXI)

in which Q, $Q_1$, $R_2$, $R_3$, $R_5$, $Z_2$, a and b are as defined previously, with a compound of formula (XXII):

$R_5COC(=X)NHR_1$ (XXII)

in which $R_5$, X and $R_1$ are defined as previously.

**17.** A pesticide composition containing a compound of formula (I) as defined in any one of claims 1 to 12, mixed with one or more supports, diluents or excipients.

**18.** A pesticide composition with synergistic effect containing a compound of formula (I) as defined in any one of claims 1 to 12, a synergist for the compound of formula (I) and one or more supports, diluents and excipients.

**19.** A mixture of one or more compounds of formula (I), as defined in any one of claims 1 to 12, and of another pesticide compound.

**20.** A method for controlling parasites, which comprises the use of an effective quantity of a compound according to any one of claims 1 to 12 or a composition or mixture according to any one of claims 17 to 19, on parasites or on the environment likely to be infested by parasites.

**21.** A compound of formula (I) according to any one of claims 1 to 12, or a composition or mixture according to any one of claims 17 to 19, for therapeutic use on the human or animal body.

**Patentansprüche**

**1.** Verbindung der Formel (I)

$$Q(CH_2)_a(O)_bQ_1CR_2=CR_3CR_4=CR_5CX_1NHR_1$$

oder eines ihrer Salze, worin Q ein System mit einem monocyclischen Kern oder ein System mit einem kondensierten bicyclischen Kern ist, die gegebenenfalls substituiert sind, worin mindestens ein Kern aromatisch ist, oder Q ist eine Dihalogenvinylgruppe oder eine Gruppe $R_6C\equiv C$-, worin $R_6$ $C_{1-4}$-Alkyl, Tri-$C_{1-4}$-Alkylsilyl, Halogen oder Wasserstoff ist;
$Q_1$ ein 1,2-Cyclopropylkern, gegebenenfalls substituiert durch eine oder mehrere Gruppe(n), ausgewählt unter $C_{1-3}$-Alkyl, Halogen, $C_{1-3}$-Halogenalkyl, $C_{1-3}$-Alkinyl oder Cyano, ist; oder $Q^1$ ist $(CH_2)_m$, worin m = 1 bis 7; a = 0 oder 1; b = 0 oder 1, ist;
$R_2$, $R_3$, $R_4$ und $R_5$ identisch oder verschieden sind, wobei eines davon wenigstens Wasserstoff ist und die anderen unabhängig voneinander ausgewählt sind unter Wasserstoff, Halogen, $C_{1-4}$-Alkyl oder $C_{1-4}$-Halogenalkyl;
$X_1$ Sauerstoff oder Schwefel ist;
$R_1$ ein heterocyclischer Kern ist, gegebenenfalls aromatisch substituiert, teilweise gesättigt oder gesättigt, mit 5 oder 6 Kettengliedern, enthaltend mindestens ein Stickstoffatom und gegebenenfalls bis zu drei andere Heteroatome, ausgewählt unter Stickstoff, Sauerstoff oder Schwefel, und der an den Stickstoff der Amidfunktion durch ein Stickstoffatom oder durch einen Kohlenstoff, der einem Stickstoffatom benachbart ist, gebunden ist, oder $R_1$ ist der 1-Azepinylrest.

**2.** Verbindung der Formel (I) oder eines ihrer Salze gemäß Anspruch 1, worin Q eine Phenyl-, Pyridyl-, Thienyl-, Naphthyl-, Chinoleinyl-, Tetrahydronaphthyl- oder Indanylgruppe ist, gegebenenfalls jeweils substituiert durch
a) einen $C_{1-6}$-Kohlenwasserstoffrest, $C_{1-6}$-Alkoxy- oder Methylendioxyrest, gegebenenfalls jeweils substituiert durch 1 bis 5 Halogenatome; oder
b) ein Halogenatom, einen Cyano-, Nitrorest; oder
c) eine Gruppe $S(O)_nR_7$, worin n = 0, 1 oder 2, und $R_7$ ist $C_{1-6}$-Alkyl, gegebenenfalls substituiert durch ein oder mehrere Halogenatome, oder $R_7$ ist Amino, gegebenenfalls substituiert durch eine oder zwei Gruppe(n) $C_{1-6}$-Alkyl; oder
d) eine Gruppe $NR_8R_9$, worin $R_8$ und $R_9$ unabhängig voneinander ausgewählt sind unter Wasserstoff, $C_{1-6}$-Alkyl oder eine Gruppe $COR_{12}$, worin $R_{12}$ $C_{1-6}$-Alkyl oder $C_{1-6}$-Alkoxy ist.

**3.** Verbindung der Formel (I) oder eines ihrer Salze gemäß Anspruch 1 oder 2, worin $R_1$ eine Pyrrol-, Pyrazol-, Imidazol-, Thiazol-, Oxazol-, Isoxazol-, Isothiazol-, Triazol-, Piperidin-, Morpholingruppe ist, gegebenenfalls jeweils substituiert durch 1 bis 5 Substituenten, ausgewählt unter: $C_{1-4}$-Alkyl und $C_{1-4}$-

Alkoxy, gegebenenfalls jeweils substituiert durch 1 bis 5 Halogenatome; Halogen, Cyano, $C_{1-3}$-Alkinyl, $C_{1-3}$-Alkenyl, Nitro, eine Gruppe $S(O)_nR_7$, worin n = 0, 1 oder 2, und $R_7$ ist $C_{1-4}$-Alkyl, gegebenenfalls substituiert durch 1 bis 5 Halogene, eine Gruppe $NR_8R_9$, worin $R_8$ und $R_9$ individuell ausgewählt sind unter Wasserstoff oder $C_{1-4}$-Alkyl, eine Gruppe $=X_2$, worin $X_2$ 0, S oder $NR_{10}$ (worin $R_{10}$ ausgewählt ist unter Wasserstoff, $C_{1-4}$-Alkyl, $C_{1-4}$-Alkoxy und $COR_{11}$, worin $R_{11}$ $C_{1-4}$-Alkyl ist) bedeutet.

**4.** Verbindung der Formel (I) oder eines ihrer Salze gemäß einem der Ansprüche 1 bis 3, worin unabhängig voneinander $R_2$ Wasserstoff bedeutet; $R_3$ Wasserstoff oder Fluoro bedeutet; $R_5$ Wasserstoff oder Fluoro bedeutet; und $R_4$ bedeutet Wasserstoff oder $C_{1-4}$-Alkyl.

**5.** Verbindung der Formel (I) oder eines ihrer Salze gemäß einem der Ansprüche 1 bis 4, worin $Q_1$ eine 1,2-Cyclopropylgruppe ist, die in den 2- und 3-Stellungen nicht substituiert ist, und substituiert oder nicht substituiert durch Fluoro oder Chloro in der 1-Stellung ist; b = 0; und a = 0.

**6.** Verbindung der Formel (I) oder eines ihrer Salze gemäß einem der Ansprüche 1 bis 4, worin $Q_1$ eine Gruppe $(CH_2)_m$ ist; b = 1; m = 6, wenn a = 1, oder m = 7, wenn a = 0.

**7.** Verbindungen der Formel (I) oder eines ihrer Salze gemäß Anspruch 1, entsprechend einer Verbindung der Formel (II)

$$Q_aQ_{1a}CR_{2a} = CR_{3a}CR_{4a} = CR_{5a}C(=X_{1a})\ NHR_{1a} \qquad (II)$$

worin $Q_a$ eine gegebenenfalls substituierte Phenyl- oder Pyridylgruppe ist, oder ein gegebenenfalls substituiertes, bicyclisches System mit kondensiertem Kern, wovon wenigstens ein Kern aromatisch ist, und enthaltend 0 oder 1 Stickstoffatom oder 0 oder 1 Schwefelatom;
$Q_{1a}$ ein 1,2-Cyclopropylkern ist, gegebenenfalls substituiert durch eine oder mehrere Gruppen, ausgewählt unter $C_{1-3}$-Alkyl, Halogen oder $C_{1-3}$-Halogenalkyl;
$R_{2a}$, $R_{3a}$, $R_{4a}$ und $R_{5a}$ identisch oder verschieden sind, wobei wenigstens eines davon Wasserstoff ist und die anderen individuell ausgewählt sind unter Wasserstoff, Halogen, $C_{1-4}$-Alkyl und $C_{1-4}$-Halogenalkyl;
$X_{1a}$ Sauerstoff oder Schwefel ist;
und $R_{1a}$ wie in Anspruch 1 für $R_1$ definiert ist.

**8.** Verbindungen der Formel (II) gemäß Anspruch 7, worin $Q_a$ eine Phenyl-, Pyridyl- oder Naphthylgruppe ist, gegebenenfalls jeweils substituiert durch
a) $C_{1-6}$-Alkyl, $C_{1-6}$-Alkoxy oder Methylendioxy, gegebenenfalls jeweils substituiert durch ein oder mehrere Halogenatom(e); oder
b) Halogen, Cyano, Nitro; oder
c) eine Gruppe $S(O)_nR_{7a}$ ist, worin n = 0, 1 oder 2, und $R_{7a}$ ist $C_{1-6}$-Alkyl, gegebenenfalls substituiert durch ein Halogenatom.

**9.** Verbindungen der Formel (II) oder eines ihrer Salze gemäß Anspruch 7 oder gemäß Anspruch 8, entsprechend einer Verbindung der Formel (III)

$$Q_aQ_{1a}CH=CHCR_{4a}=CHCONHR_{1a} \qquad (III)$$

worin $Q_a$ wie in Anspruch 7 oder in Anspruch 8 definiert ist, und $Q_{1a}$, $R_{4a}$ und $R_{1a}$ wie in Anspruch 7 definiert sind.

**10.** Verbindungen der Formel (I) oder eines ihrer Salze gemäß Anspruch 1, entsprechend einer Verbindung der Formel (IV)

$$Q\ Q_1CH=CR_3CR_4=CR_5CONHR_1 \qquad (IV)$$

worin Q, $Q_1$, $R_4$ und $R_1$ wie in Anspruch 1 definiert sind.

**11.** Verbindungen der Formel (IV) gemäß Anspruch 10, worin Q substituiertes Phenyl ist; $Q_1$ ein trans-1,2-Cyclopropyl-kern ist, worin die 2-Stellung des Cyclopropylkerns nicht substituiert ist oder substituiert ist

durch Fluoro oder Chloro; $R_4$ Methyl oder Wasserstoff ist; $R_3$ und $R_5$ individuell Wasserstoff oder Fluoro sind; und $R_1$ wie in Anspruch 1 definiert ist.

**12.** Verbindungen der Formel (I) gemäß Anspruch 1, ausgewählt unter:
(±)-(2E,4E)-N-(2-Pyridyl)-5-[c-2-(3,4-dibromophenyl)-r-1-fluorocyclopropyl]-3-methylpenta-2,4-dienamid ;
(±)-(2E,4E)-N-(3-Methyl-2-pyridyl)-5-[c-2-(3,4-dibromophenyl)-r-1-fluorocyclopropyl]penta-2,4-dienamid ;
(±)-(2E,4E)-N-(2-Thiazolyl)-5-[c-2-(3,4-dibromophenyl)-r-1-fluorocyclopropyl]penta-2,4-dienamid ;
(±)-(2E,4E)-N-(5-Methylisoxazole-3-yl)-5-[c-2-(3,4-dibromophenyl)-r-1-fluorocyclopropyl]penta-2,4-dienamid ;
(±)-(2E,4E)-N-(1,3,4-Thiadiazol-2-yl)-5-[c-2-(3,4-dibromonhenyl)-r-1-fluorocyclopropyl]penta-2,4-dienamid ;
(±)-(2E,4E)-N-(4-Pyrimidyl)-5-[c-2-(3,4-dibromophenyl)-r-1-fluorocyclopropyl]penta-2,4-dienamid ;
(±)-(2E,4E)-N-(3-Pyrazolyl)-5-[c-2-(3,4-dibromophenyl)-r-1-fluorocyclopropyl]penta-2,4-dienamid ;
(±)-(2E,4E)-N-(4-Trifluoromethyl-1,3-thiazolyl)-5-[c-2-(3,4-dibromophenyl)-r-1-fluorocyclopropyl]penta-2,4-dienamid ;
(±)-(2E,4E)-N-2-(4-Trifluoromethyl-5-chloro-1,3-thiazolyl)-5-[c-2-(3,4-dibronophenyl)-r-1-fluorocyclopropyl]penta-2,4-dienamid ;
(±)-(2E/Z,4E)-N-Piperidino-5-[c-2-(3,4-dibromophenyl)-r-1-fluorocyclopropyl]penta-2,4-dienamid ;
(±)-(2E,4E)-N-Piperidino-5-[c-2-(3,4-dibromophenyl)-r-1-fluorocyclopropyl]penta-2,4-dienamid ;
(±)-(2E,4E)-N-(2,6-Dimethylpipéridino)-5-[c-2-(3,4-dibromophenyl)-r-1-fluorocyclopropyl]penta-2,4-dienamid ;
(±)-(2E,4E)-N-Morpholino-5-[c-2-(3,4-dibromophenyl)-r-1-fluorocyclopropyl]penta-2,4-dienamid ;
(±)-(2E,4E)-N-(Perhydroazepin-1-yl)-5-[c-2-(3,4-dibromophenyl)-r-1-fluorocyclopropyl]penta-2,4-dienamid;
und ihre Salze.

**13.** Verfahren zur Herstellung einer Verbindung gemäß einem der Ansprüche 1 bis 12, welches umfaßt: entweder

a) wenn $X_1$ Sauerstoff ist, die Reaktion der Säure oder des entsprechenden Derivats der Säure der Formel (V)

$$Q(CH_2)_a(O)_bQ_1CR_2=CR_3CR_4=CR_5C(=X)Z_1 \qquad (V)$$

mit einem Amin $H_2NR_1$, worin Q, a, b, $Q_1$, $R_2$, $R_3$, $R_4$, $R_5$ und $R_1$ wie in Anspruch 1 definiert sind, und X Sauerstoff ist, und $Z_1$ Hydroxy, $C_{1-6}$-Alkoxy, Halogen oder Phosphoroimidatester (-P(O)(O-Aryl)NH-aryl, worin Aryl $C_{1-6}$-Aryl ist) ist; oder

b) die Bildung des Teils $-CH_2=CR_3CR_4=CR_5C(=X_1)NHR_1$ der Verbindung der Formel (I) über die Zwischenverbindung einer Reaktion vom Wittig-Typ;

und gegebenenfalls spätere Überführung einer der Verbindungen der Formel (I) in eine andere Verbindung der Formel (I) nach dem Fachmann bekannten Methoden.

**14.** Verfahren gemäß Anspruch 13, worin in a) die Verbindung der Formel (V), worin $Z_1$ für Hydroxy steht, und Q, $Q_1$, $R_2$, $R_3$, $R_4$, $R_5$, a und b wie in Anspruch 1 definiert sind, hergestellt wird durch Hydrolyse des entsprechenden Esters, worin $Z_1$ $C_{1-6}$-Alkoxy ist, und Q, $Q_1$, $R_2$, $R_3$, $R_4$ und $R_5$, a und b wie in Anspruch 1 definiert sind.

**15.** Verfahren gemäß Anspruch 14, wobei die Esterverbindung der Formel (V), worin $Z_1$ für $C_{1-6}$-Alkoxy steht, und Q, $Q_1$, $R_2$, $R_3$, $R_4$, $R_5$, a und b wie in Anspruch 1 definiert sind, hergestellt wird durch ein Verfahren, das umfaßt entweder

i) die Reaktion einer Verbindung der Formel (VI)

$$Z_2CHR_3CR_4=CR_5COZ_1 \qquad (VI)$$

worin $R_3$, $R_4$, $R_5$ und $Z_1$ wie vorstehend definiert sind, und $Z_2$ eine Gruppe $(Aryl)_3P$, $(Aryl)_2P(O)$ oder $(C_{1-4}\text{-Alkoxy})_2P(O)$ bedeutet, mit einer Verbindung der Formel (VII)

$$Q(CH_2)_a(O)_bQ_1CR_2=O \qquad (VII)$$

worin Q, $Q_1$, $R_2$, a und b wie vorstehend definiert sind; oder
ii) die Umlagerung und Entfernung von $HS(O)Z_3$ einer Verbindung der Formel (VIII)

$$Q(CH_2)_a(O)_bQ_1CHR_2CHR_3CR_4 = C \begin{matrix} / S(O)Z_3 \\ \backslash CO_2Z_4 \end{matrix} \qquad (VIII)$$

worin Q, $Q_1$, $R_2$, $R_3$, $R_4$, $Z_1$, a und b wie vorstehend definiert sind, und $Z_3$ irgendeine geeignete Gruppe ist, vorzugsweise Phenyl, substituiertes Phenyl oder $C_{1-4}$-Alkyl; oder
iii) die Entfernung von $HOZ_5$ aus einer Verbindung der Formel (IX)

$Q(CH_2)_a(O)_bQ_1CHR_2CR_3(OZ_5)CR_4 = CR_5\text{-}COZ_1$ (IX)

worin Q, $Q_1$, $R_2$, $R_3$, $R_4$, $R_5$, $Z_1$, a und b wie vorstehend definiert sind, und $Z_5$ Wasserstoff oder $C_{1-4}$-Acyl ist; oder
(iv) die Reaktion einer Verbindung der Formel (X)

$Q(CH_2)_a(O)_bQ_1CR_2 = CR_3C(=O)R_4$ (X)

worin Q, $Q_1$, $R_2$, $R_3$, $R_4$, a und b wie vorstehend definiert sind, mit einer Verbindung der Formel (XI)

$Me_3SiCHR_5COZ_1$ (XI)

worin $R_5$ und $Z_1$ wie vorstehend definiert sind; oder
v) Reaktion einer Verbindung der Formel (XII)

$Q(CH_2)_a(O)_bQ_1CR_2 = CR_3C(OZ_6) = CR_5COZ_1$ (XII)

worin Q, $Q_1$, $R_2$, $R_3$, $R_5$, $Z_1$, a und b wie vorstehend definiert sind, und $Z_6$ irgendeine geeignete Gruppe ist, vorzugsweise eine Dialkylphosphat- oder Trifluormethansulfonatgruppe, mit einer Verbindung der Formel (XIII)

$R^4M^1$ (XIII)

worin $R_4$ wie vorstehend definiert ist, und M ein Metall ist, vorzugsweise Kupfer-(I) oder Kupfer-(II), assoziiert mit Lithium oder Magnesium; oder
vi) die Reaktion einer Verbindung der Formel (XIV)

$Q(CH_2)_a(O)_bQ_1CR_2 = CR_3M_2$ (XIV)

worin Q, $Q_1$, $R_2$, $R_3$, a und b wie vorstehend definiert sind, und $M_2$ eine Gruppe ist, enthaltend Silyl, vorzugsweise Trimethylsilyl, oder eine Gruppe, enthaltend ein Metall, vorzugsweise eine Gruppe, enthaltend Zirkonium, Zinn, Aluminium oder Zink, noch bevorzugter eine Bis-(cyclopentadienyl)-zirkonium-chloridgruppe, mit einer Verbindung der Formel (XV)

$YCR_4 = CR_5COZ_1$ (XV)

worin $R_4$, $R_5$ und $Z_1$ wie vorstehend definiert sind, und Y Halogen oder Zinn ist; oder

vii) die Entfernung von $Z_3S(O)H$ aus einer Verbindung der Formel (XVI)

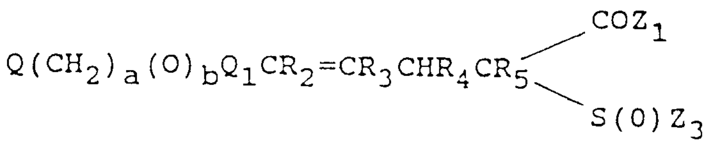

(XVI)

worin Q, $Q_1$, $R_2$, $R_3$, $R_4$, $R_5$, $Z_1$, a, b und $Z_3$ wie vorstehend definiert sind.

**16.** Verfahren gemäß Anspruch 13, worin in b) die Verbindung der Formel (I), worin Q, $Q_1$, $R_2$, $R_3$, $R_4$, $R_5$, a, b, $R_1$ und X wie in Anspruch 1 definiert sind, hergestellt wird durch ein Verfahren, das umfaßt entweder

i) Reaktion einer Verbindung der Formel (XVII)

$Q(CH_2)_a(O)_bQ_1CR_2=CR_3CR_4=O$ (XVII)

worin Q, $Q_1$, $R_2$, $R_3$, $R_4$, a und b wie vorstehend definiert sind, mit einer Verbindung der Formel (XVIII)

$Z_2CHR_5C(=X)NHR_1$ (XVIII)

worin $R_1$, X und $R_5$ wie vorstehend definiert sind, und $Z_2$ eine Gruppe $(Aryl)_3P$, $(Aryl)_2P(O)$ oder $(C_{1-4}\text{-Alkoxy})_2P(O)$ ist; oder

ii) Reaktion einer Verbindung der Formel (XIX)

$Q(CH_2)_a(O)_bQ_1COR_2$ (XIX)

worin Q, $Q_1$, $R_2$, a und b wie vorstehend definiert sind, mit einer Verbindung der Formel (XX)

$Z_2CHR_3CR_4=CR_5C(=X)NHR_1$ (XX)

worin $Z_1$, $R_3$, $R_4$, $R_5$, X und $R_1$ wie vorstehend definiert sind; oder

iii) Reaktion einer Verbindung der Formel (XXI)

$Q(CH_2)_a(O)Q_{1b}CR_2=CR_3CHR_5Z_2$ (XXI)

worin Q, $Q_1$, $R_2$, $R_3$, $R_5$, $Z_2$, a und b wie vorstehend definiert sind, mit einer Verbindung der Formel (XXII)

$R_5COC(=X)NHR_1$ (XXII)

worin $R_5$, X und $R_1$ wie vorstehend definiert sind.

**17.** Pestizide Zusammensetzung, enthaltend eine Verbindung der Formel (I), wie in einem der Ansprüche 1 bis 12 definiert, im Gemisch mit einem oder mehreren Trägern, Verdünnungsmitteln oder Exzipienten.

**18.** Pestizide Zusammensetzung mit synergistischer Wirkung, enthaltend eine Verbindung der Formel (I), wie in einem der Ansprüche 1 bis 12 definiert, einen Synergist für die Verbindung der Formel (I) und einen oder mehrere Träger, Verdünnungsmittel und Exzipienten.

**19.** Gemisch aus einer oder mehreren Verbindungen der Formel (I), wie in einem der Ansprüche 1 bis 12 definiert, und einer weiteren pestiziden Verbindung.

**20.** Verfahren zur Bekämpfung von Parasiten, welches die Anwendung einer wirksamen Menge einer Verbindung gemäß einem der Ansprüche 1 bis 12 umfaßt oder eine Zusammensetzung oder ein Gemisch gemäß einem der Ansprüche 17 bis 19, auf Parasiten oder auf die für den Parasitenbefall

empfindliche Umgebung.

**21.** Verbindung der Formel (I) gemäß einem der Ansprüche 1 bis 12 oder eine Zusammensetzung oder ein Gemisch gemäß einem der Ansprüche 17 bis 19 zur Verwendung in der Therapie, die auf dem menschlichen oder tierischen Körper eingesetzt wird.